(19) Europäisches Patentamt
European Patent Office
Office européen des brevets

(11) EP 0 611 755 B1

(12) **FASCICULE DE BREVET EUROPEEN**

(45) Date de publication et mention de la délivrance du brevet:
**10.07.1996 Bulletin 1996/28**

(51) Int Cl.[6]: **C07C 403/20**, C07C 403/18, C07F 7/18

(21) Numéro de dépôt: **94400296.3**

(22) Date de dépôt: **11.02.1994**

(54) **Nouveaux composés (cyclohexyl) alcéniques, leurs procédés de préparation et les compositions pharmaceutiques qui les contiennent**

(Cyclohexyl)alkenderivate, Verfahren zu deren Herstellung und diese enthaltende pharmazeutische Zusammensetzungen

(Cyclohexyl)-alken compounds, process for their preparation and pharmaceutical compositions containing them

(84) Etats contractants désignés:
**AT BE CH DE DK ES FR GB GR IE IT LI LU NL PT SE**

(30) Priorité: **16.02.1993 FR 9301690**

(43) Date de publication de la demande:
**24.08.1994 Bulletin 1994/34**

(73) Titulaire: **ADIR ET COMPAGNIE**
**F-92415 Courbevoie Cédex (FR)**

(72) Inventeurs:
- **Girard, Jean-Pierre**
**F-34000 Montpellier (FR)**
- **Hullot, Pierre**
**F-34660 Cournonterral (FR)**
- **Bonne, Claude**
**F-34000 Montpellier (FR)**
- **Rossi, Jean-Claude**
**F-34000 Montpellier (FR)**
- **Escale, Roger Villa Rieumassel**
**F-34790 Grabels (FR)**
- **Muller, Agnès**
**F-34970 Lattes (FR)**

(56) Documents cités:
**US-A- 4 090 019**

EP 0 611 755 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux composés (cyclohexyl)alcéniques, leurs procédés de préparations, et les compositions pharmaceutiques qui les contiennent.

Le leukotriène $B_4$ ($LTB_4$) est un acide icosatétraénoïque dihydroxylé issu du métabolisme de l'acide arachidonique. Il est produit par de nombreuses cellules telles que les neutrophiles, les monocytes et les macrophages. Le $LTB_4$ est connu pour être un acteur important de l'activation de nombreuses fonctions des neutrophiles. Il stimule l'agrégation et la dégranulation des neutrophiles humains, il induit le chimiotactisme des leukocytes et est un promoteur de la génération des radicaux superoxide.

Par conséquent, le $LTB_4$ apparaît comme un important médiateur de l'inflammation.

Chez l'homme, le $LTB_4$ a été notamment détecté dans le liquide synovial rhumatoïde, dans les fluides arthritiques de malades atteints de goutte, dans les muqueuses gastrointestinales inflammées et dans la peau des personnes atteintes de psoriasis (Critical Rewiews in Immunology 1990, vol. 10 (1), p 1-12).

Les neutrophiles et de nombreuses autres cellules possèdent des récepteurs membranaires spécifiques au $LTB_4$, qui médient la plupart des réponses de ces cellules au $LTB_4$.

Des antagonistes spécifiques des récepteurs du $LTB_4$ seraient donc utiles pour le clinicien dans le traitement de nombreuses conditions ou maladies inflammatoires où le $LTB_4$ intervient comme facteur pathologique.

La demanderesse a découvert de nouveaux composés (cyclohexyl)alcéniques qui sont de puissants antagonistes des récepteurs $LTB_4$.

Plus particulièrement, l'invention concerne les composés de formule (I) :

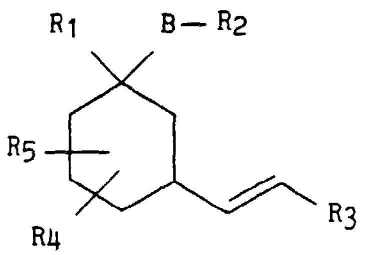

(I)

dans laquelle :

- B représente une chaine alkylène de 1 à 8 atomes de carbone non substituée ou substituée par 1 ou 2 radicaux alkyles,
- $R_1$ représente un atome d'hydrogène ou un radical choisi parmi hydroxy et alkoxy,
- $R_2$ représente un radical choisi parmi :
  - $CH_2$ - OH et

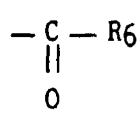

dans lequel $R_6$ représente un groupement choisi parmi hydroxyle, alkoxy, et

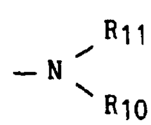

dans lequel $R_{10}$ et $R_{11}$ représentent, chacun indépendamment l'un de l'autre, un atome d'hydrogène, un radical alkyle, ou un groupement aryle ou arylalkyle, le groupement aryle ou arylalkyle étant non substitué ou substitué sur le noyau aryle par un ou plusieurs radicaux choisis parmi halogène, alkyle, alkoxy et trifluorométhyle, ou bien $R_{10}$ et $R_{11}$ forment ensemble avec l'atome d'azote qui les porte un hétérocycle choisi parmi pyrrolidine, pipéridine, pipérazine, morpholine, et thiomorpholine ;

- $R_3$ représente un groupement de formule :

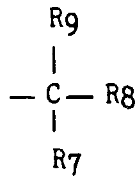

dans lequel :

- $R_7$ représente un radical choisi parmi hydroxy, alkoxy, et -O-$R_A$ dans lequel $R_A$ représente un acyle ou un groupement protecteur de la fonction hydroxyle,
- $R_9$ représente un atome d'hydrogène ou un radical choisi parmi alkyle, aryle, et arylakyle, le groupement aryle ou arylalkyle étant non substitué ou substitué sur le noyau aryle par un ou plusieurs radicaux choisis parmi halogène, alkyle, alkoxy et trifluorométhyle,
- ou bien $R_7$ et $R_9$ forment un groupement oxo,
- et $R_8$ représente un groupement alkyle linéaire ou ramifié de 1 à 12 atomes de carbone non substitué ou substitué par un ou plusieurs radicaux choisis parmi :
  - . hydroxyle,
  - . halogène,
  - . aryle non substitué,
  - . aryle substitué par un ou plusieurs radicaux choisis parmi halogène, hydroxy, alkyle, alkoxy, et trifluorométhyle,
  - . cycloalkyle non substitué,
  - . cycloalkyle substitué par un ou plusieurs radicaux choisis parmi halogène, alkyle, oxo, et alkoxy,
- $R_8$ pouvant éventuellement contenir dans son squelette carboné :
  - un ou deux hétéroatomes choisis parmi azote, soufre, et oxygène,
  - une ou plusieurs liaisons insaturées,
- $R_4$ et $R_5$ représentent chacun indépendamment l'un de l'autre, un radical choisi parmi hydrogène et alkyle, ou bien, $R_4$ et $R_5$ forment ensemble avec le cyclohexane qui les porte un groupement 1,2,3,4-tetrahydronaphtalène ou un groupement perhydronaphtalène, étant entendu que lors de la description de la formule (I), et sauf précisions contraires :
- les termes "alkyle", "alkoxy" et "acyle" désignent des groupements linéaires ou ramifiés contenant de 1 à 6 atomes de carbone,
- le terme "aryle" désigne un groupement phényle ou naphtyle,
- le terme "liaison insaturée" désigne une double ou une triple liaison,
- le terme "cycloalkyle" désigne un groupement de 3 à 7 atomes de carbone,

leurs énantiomères et diastéréoisomères,
leurs isomères Z et E,
et leurs sels d'addition à un acide ou à une base pharmaceutiquement acceptable.
De façon particulière, les radicaux alkyles présents dans les différents substituants de la formule (I) peuvent être choisis parmi méthyle, éthyle, n-propyle, isopropyle, n-butyle, isobutyle, sec-butyle, tert-butyle, pentyle ou n-pentyle, hexyle ou n-hexyle, ainsi que, dans le cas de $R_8$, également parmi heptyle, n-heptyle, octyle, n-octyle, nonyle, n-nonyle, décyle, n-décyle, undécyle, n-undécyle, dodécyle, et n-dodécyle.

Les radicaux alcoxy présents dans les substituants de la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans les substituants de la formule (I) peuvent être choisis parmi le brome, le chlore, le fluor, et l'iode.

Les cycloalkyles présents dans les substituants de la formule (I) peuvent être choisis parmi cyclopropyle, cyclobutyle, cyclopentyle, cyclohexyle, et cycloheptyle.

Les groupes O-protecteurs éventuellement présents dans le substituant $R_7$ sont les groupes O-protecteurs connus de l'homme de l'art et de préférence ceux qui sont éliminables par hydrolyse acide douce, tels que les groupes tert-butyldiméthylsilyl, tetrahydropyran-2-yle, et méthoxy-méthyle, méthyldiphénylsilyl, et tert-butyldiphénylsilyl.

Les groupements B présents dans la formule (I) peuvent être choisis parmi méthylène, éthylène, triméthylène, tétraméthylène, pentaméthylène, hexaméthylène, heptaméthylène, et octaméthylène.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

L'invention s'étend également au procédé de préparation des composés de formule (I) caractérisé en ce que : on fait réagir un composé de formule (II) :

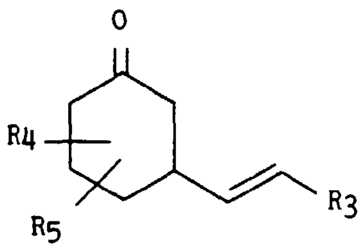

**(II)**

dans laquelle $R_3$, $R_4$, et $R_5$ sont tels que décrits dans la formule (I) avec un composé de formule (III) :

$$^{\ominus}B - R_2 \qquad (III)$$

dans laquelle $R_2$ et B sont tels que décrits dans la formule (I) pour obtenir le composé de formule (I/a) correspondant :

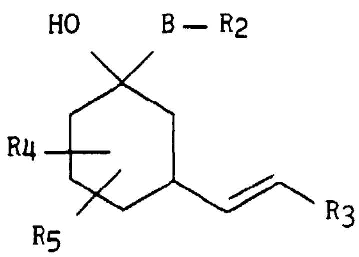

**(I/a)**

dans laquelle $R_2$, $R_3$, $R_4$, $R_5$ et B sont tels que définis précédemment, qui peuvent être, si on le désire,

- soit soumis à l'action d'un composé de formule (IV) :

$$R'^{\oplus} \qquad (IV)$$

dans laquelle R' représente un alkyle pour obtenir les composés de formule ($I_b$) :

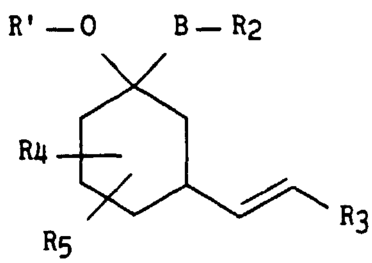

**($I_b$)**

dans laquelle $R_2$, $R_3$, $R_4$, $R_5$, R' et B sont tels que définis précédemment,

- soit soumis au chlorure de tosyle puis à une réduction par l'hydrure d'aluminium et de lithium pour conduire au composé de formule ($I_c$) :

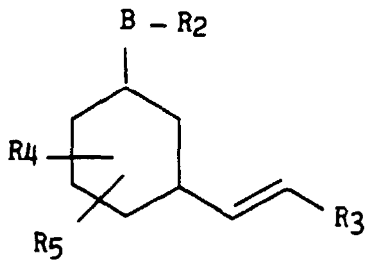

(Ic)

dans laquelle $R_2$, $R_3$, $R_4$, $R_5$ et B sont tels que définis précédemment,
les composés de formule (I/a), (I/b), et (I/c) dans lesquels $R_9$ représente un atome d'hydrogène et $R_7$ représente un radical hydroxyle pouvant être oxydé afin d'obtenir les composés de formule (I/d) :

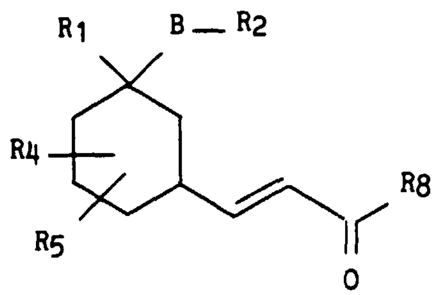

(I/d)

dans laquelle R1, R2, R4, R5, R8, et B sont tels que précédemment,

les composés de formule (I/a), (I/b), (I/c), et (I/d) représentant l'ensemble des composés de formule (I), composés de formule (I) qui sont, si on le désire, séparés en leurs différents énantiomères ou diastéréoisomères, et salifiés le cas échéant avec un acide ou une base pharmaceutiquement acceptable.

Les matières premières utilisées dans le procédé précédemment décrit sont soit commerciales, soit aisément accessibles, à l'homme de l'art selon des procédés connus dans la littérature ou proposés lors des exemples de préparations décrits ci-après dans cette demande.

Les composés de formule (II) sont aisément accessibles à l'homme de l'art par réaction d'un composé de formule (II/a) :

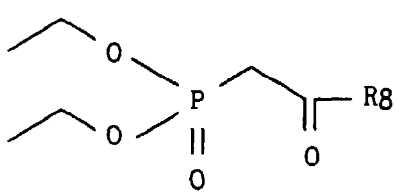

(II/a)

dans laquelle $R_8$ est telle que décrit dans la formule (I) (obtenu selon la méthode de SAVIGNAC P. et al, Tetrahedron Letters, 1976, pp 2829-2832) avec un composé de formule (II/b) :

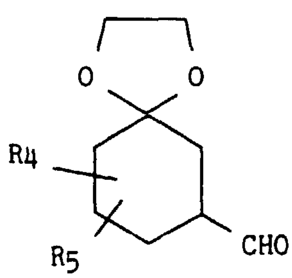

(II/b)

dans laquelle $R_4$ et $R_5$ sont tels que définis dans la formule (I), pour obtenir (selon la méthode de TAKACS J.M. et al. Tetrahedron Letters, 1986, vol. 27, pp 1257-1260) un composé de formule (II/c) :

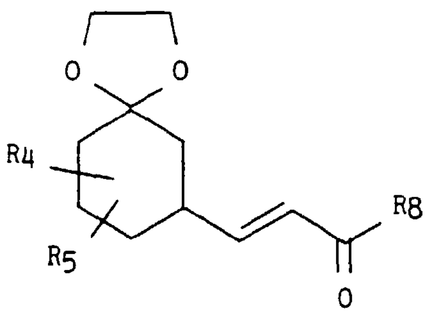

(II/c)

dans laquelle R4, $R_5$ et $R_8$ sont tels que définis précédemment,
composé de formule (II/c) qui est soit réduit, le cas échéant de façon énantiosélective, pour obtenir un composé de formule (II/d) :

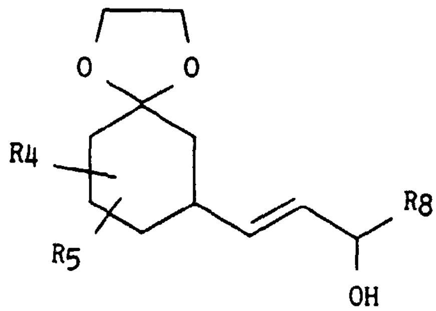

(II/d)

dans laquelle $R_4$, $R_5$ et $R_8$ sont tels que définis précédemment, qui est ensuite déprotégé, après une éventuelle alkylation de la fonction hydroxyle, soit soumis à l'action d'un nucléophile $R_9$ avec $R_9$ tel que défini dans la formule (I), pour obtenir, après déprotection et un éventuelle alkylation de la fonction hydroxy, le composé de formule (II/e) :

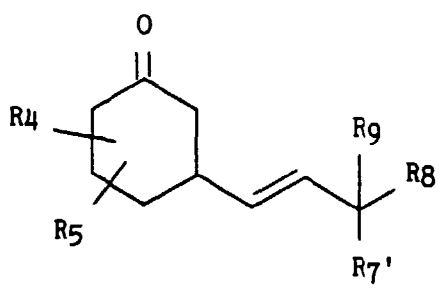

(II/e)

dans laquelle $R_4$, $R_5$, $R_8$ et $R_9$ sont tels que définis précédemment et $R_7$' représente un radical hydroxy ou alkoxy, composé de formule (II/e) dont, la fonction hydroxyle représentée, le cas échéant, par $R_7$', peut être acylée ou protégée par greffage d'un groupement O-protecteur pour donner un composé de formule (II/f) :

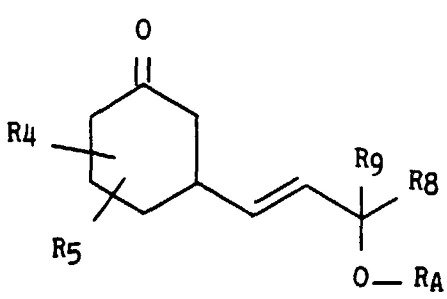

(II/f)

dans laquelle $R_4$, $R_5$, $R_8$ et $R_9$ sont tels que définis précédemment, et $R_A$ est tel que défini dans la formule (I),
les composés de formule (II/e) et (II/f) formant l'ensemble des composés de formule (II).

L'invention s'étend également au procédé de préparation des composés de formule (I) caractérisé en ce que la séparation des diastéréoisomères se réalise par chromatographie sur colonne.

L'invention s'étend également au procédé de préparation des composés de formule (I) caractérisé en ce que la séparation des diastéréoisomères se réalise par chromatographie sur colonne et en ce que le groupement $R_7$ des composés de formule (I) représente un groupement tert-butyldiméthylsilyloxy.

La demanderesse a découvert que les composés de l'invention possédaient une remarquable activité antagoniste du $LTB_4$. Ils présentent notamment une très haute affinité pour les récepteurs du $LTB_4$.

Cette capacité de liaison très importante est mise en évidence dans l'exemple A du présent document (Etude de liaison des composés de l'invention aux récepteurs du $LTB_4$).

La forte activité antagoniste du $LTB_4$ révélée par les composés de l'invention est démontrée aux exemples B et C du présent document (Exemple B : Etude de l'activité des composés de l'invention sur le chimiotactisme des PMN humains in vitro et Exemple C : Etude de l'activité des composés de l'invention sur la contraction de bandelettes de poumon).

Les composés de l'invention apparaissent donc comme de puissants antagonistes des récepteurs du $LTB_4$ et des mécanismes pathologiques dont le $LTB_4$ est le médiateur.

De par leur action, les composés de l'invention sont donc de nouveaux candidats pour le traitement et la prévention des maladies inflammatoires et des conditions inflammatoires pathologiques.

Les composés de l'invention sont par conséquent utiles dans le traitement et la prévention de l'inflammation chronique ou aiguë, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux, de l'ischémie, de l'athérosclérose, de la détresse respiratoire, et du choc septique.

La présente invention a également pour objet les compositions pharmaceutiques contenant un des composés de formule (I), ou un de ses sels d'addition à un acide ou à une base pharmaceutiquement acceptable, seul ou en combinaison avec un ou plusieurs excipients inertes non toxiques.

Parmi les compositions pharmaceutiques selon l'invention, on pourra citer, à titre d'exemples et de façon non limitative, celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les suppositoires, les crèmes, pommades, et les gels dermiques.

La posologie utile varie selon l'âge et le poids du patient, la voie d'administration, la nature de l'affection et des traitements éventuellement associés et s'échelonne entre 0,1 mg et 100 mg par 24 heures en 1 à 2 prises.

Les préparations suivantes décrites sont utiles dans la synthèse des composés de l'invention. Elles ne font pas partie de l'invention.

**DONNEES TECHNIQUES** :

CHROMATOGRAPHIE :

- Chromatographie sur couche mince (CCM) : gel de silice 60 $F_{254}$ (Merck)
- Chromatographie sur colonne ouverte : silice Matrex, 60 Å, 70-200 mesh silice Matrex, 60 Å, 35-70 mesh (Société Amicon)
- Chromatographie en phase vapeur (CPV) :

  . Appareil Delsi 30

  Injecteur split-splitless (rapport de division : 1/20).
  Détecteur à ionisation de flamme d'hydrogène.
  Colonne capillaire silice fondue (1 = 30 m, Ø = 0,32 mm, film de 1 µ DB5).
  Gaz vecteur : hélium, débit 2cm$^3$/min.
  Intégrateur-calculateur : Enika 21.
  Ti : température d'injection.
  program : programmation de la température du four en °C/min.
  tr : temps de rétention en secondes.

POINT DE FUSION :

Les points de fusion (F) sont pris dans des tubes capillaires sur un appareil BUCHI TOTTOLI et ne sont pas corrigés.

INFRA ROUGE :

Les spectres infra-rouge (IR) sont enregistrés sur un appareil BECKMANN ACCU-LAB-2, en films sur cellules de chlorure de sodium (NaCl) pour les liquides, ou en pastilles de bromure de potassium (KBr) pour les solides.

RESONNANCE MAGNETIQUE NUCLEAIRE (RMN)

Les spectres de résonnance magnétique nucléaire du proton ($RMN^1H$) sont enregistrés sur un appareil VARIAN EM 360 A à 60 MHz (référence : tétraméthylsilane) et sur un appareil BRUKER WH 360 WB à 360 MHz. Les spectres de corrélation $^1H$-$^1H$ (COSY) sont enregistrés sur un appareil BRUKER WH 360 WB à 360 MHz.

Les spectres de résonnance magnétique nucléaire du phosphore ($RMN^{31}P$) sont enregistrés découplés sur un appareil BRUKER WP 2000 SY à 81,015 MHz (référence : acide phosphorique, étalon externe).

Les valeurs des déplacements chimiques (δ) sont données en partie par million (ppm), et celles des constantes de couplage (J) en Hertz.

**PREPARATION 1 : 3-((E)-3-TERT-BUTYLDIMETHYLSILYLOXY-7-PHENYL-HEPT-1-EN-1-YL)CYCLOHEXANONE**

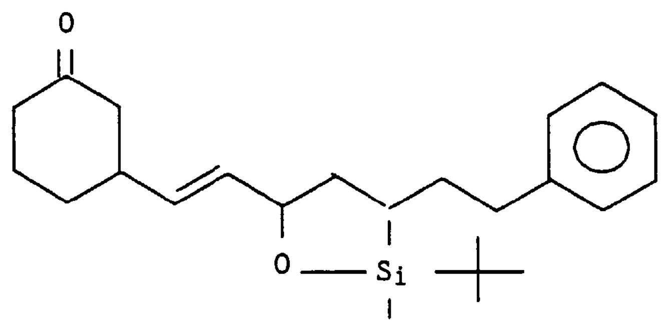

**STADE A : 3-HYDROXY CYCLOHEXANE CARBOXYLATE DE METHYLE**

Une solution de 100 g (658 mmol) de 3-hydroxy benzoate de méthyle dans 280 $cm^3$ d'éthanol est hydrogénée en présence de 2,75 g de ruthénium sur charbon à 5 %, sous 100 bars à 120° C, pendant 16 à 18 h.
Après refroidissement la solution est filtrée sur célite, le catalyseur est lavé deux fois par 50 $cm^3$ d'éther. Les solvants sont éliminés au rotavapor, le résidu est distillé sous pression réduite, on obtient 96,7 g (612 mmol) de 3-hydroxy cyclohexane carboxylate de méthyle.
Rendement : 93 %

- Eb (Point d'ébullition) (1,33 Pa) : 78°C
- Caractéristiques spectrales :
- I.R. (Infrarouge) (film, $cm^{-1}$) : 3400 (νOH) ; 1720 (νC = O)
- RMN $^1H$ ($CCl_4$)δ : 0,75 à 3 (m, 11H, H cyclohexyle et OH) ; 3,66 (s, 3H, $OCH_3$)

**STADE B : 3-OXO-CYCLOHEXANE CARBOXYLATE DE METHYLE**

A une solution de 58,3 g (369 mmol) de 3-hydroxy cyclohexane carboxylate de méthyle dans 600 $cm^3$ d'acétone pur, on ajoute goutte à goutte, en 45 min et en agitant entre + 5 et + 10°C, une solution contenant 36,9 g (369 mmol) d'anhydride chromique, 74 $cm^3$ d'eau distillée et 21 $cm^3$ d'acide sulfurique concentré. Le milieu, immédiatement brun rouge, est agité 3h à température ambiante. On ajoute 1 $cm^3$ d'acide sulfurique et quelques $cm^3$ d'isopropanol pour faire précipiter les sels de chrome en solution, on observe une décoloration progressive du milieu. Les sels de chrome sont filtrés sur célite et lavés à l'acétone. Le filtrat est agité 1 h avec de l'hydrogénocarbonate de sodium puis concentré au rotavapor. Le résidu de couleur verte est repris par 500 $cm^3$ d'éther et par 100 $cm^3$ d'une solution saturée de chlorure de sodium. Après décantation la couche aqueuse est extraite à l'éther. Les phases organiques réunies sont lavées par une solution saturée de chlorure de sodium puis séchées sur sulfate de sodium. Après concentration, on obtient 47,2 g (302 mmol) de 3-oxo-cyclohexane carboxylate de méthyle qui est soit utilisé tel quel, soit distillé sous pression réduite.
Rendement : 82 %

- Eb (5,32 $P_a$) : 77-80°C
- I.R (film, $cm^{-1}$) : 1720 (νC = O)
- RMN $^1H$ ($CCl_4$)δ : 1,3 à 3 (m, 9H, H cyclo) ; 3,66 (s, 3H, $OCH_3$)

-

| Analyse centésimale : $C_8H_{12}O_3$, M = 156, 184 | | | | |
|---|---|---|---|---|
| Calc. % : | C | 61,52 | H | 7,74 |
| Mesuré : | | 61,78 | | 8,00 |

**STADE C : 3,3-ETHYLENEDIOXY-CYCLOHEXANE CARBOXYLATE DE METHYLE**

Dans un tricol d'un litre muni d'un Dean-Stark, on introduit une solution composée de 30 g (192 mmol) de 3-oxo-cyclohexane carboxylate de méthyle brut et de 430 $cm^3$ de toluène puis 14,3 g (230 mmol) d'éthylèneglycol sont ajoutés avec 814 mg (4,3 mmol) d'acide para-toluène sulfonique monohydrate. Le mélange est chauffé à reflux, l'azéotrope toluène-eau est instantané, au bout de 40 min, il n'y a plus de reflux d'azéotrope (95°C). On maintient 6 h aux reflux du toluène (110°C). Après refroidissement on ajoute une solution d'hydrogénocarbonate de sodium puis la solution est lavée par une solution saturée de chlorure de sodium et séchée sur sulfate de sodium.
Après concentration, on obtient 39 g d'une huile jaune qui est soit distillée soit chromatographée sur une colonne de 215 g de silice (éluant cyclohexane/éther 98/2).
Remarque : la chromatographie est préférable à la distillation qui déprotège partiellement la cétone.
Rendement : 95 %

- $E_b$ (2660 Pa) : 142°C ; $E_b$ (1,33 Pa) : 64-66°C
- I.R (film, $cm^{-1}$) : 1700 (νC = 0) ; 1040-1020 (νC - 0 dioxolane)
- RMN $^1H$ ($CCl_4$)δ : 1 à 2,15 (m, 8H, $CH_2$ cyclo) ; 2,2 à 2,9 (m, 1H, CH cyclo) ; 3,63 (s, 3H, $OCH_3$) ; 3,88 (s, 4H, O-$CH_2$-$CH_2$-O)
-

| Analyse centésimale : $C_{10}H_{16}O_4$ M = 200, 237 | | | | |
|---|---|---|---|---|
| Calc. % : | C | 59,98 | H | 8,05 |
| Mesuré : | | 60,16 | | 8,06 |

Référence HOUSE, H.O. et al J. Org. Chem, 1966, vol. 31 (8), p 2667-9.

**STADE A' : METHYLPHOSPHONATE DE DIETHYLE**

Dans un tricol sec surmonté d'un réfrigérant à alcool (-10°C), 188g (1,32 mol) d'iodure de méthyle sont versés sous azote puis chauffés à température de reflux. 168g (1,02 mol) de triéthyl phosphite sont alors ajoutés goutte à goutte. La réaction est exothermique, la température s'élève en une heure jusqu'à 73°C. La température du mélange est maintenue pendant une heure entre 70 et 75°C, puis la solution est refroidie et concentrée à l'évaporateur rotatif. Le méthylphosphonate de diéthyle est distillé sous azote, sous pression réduite.
Rendement : 95 %

- Eb (2660 Pa) = 88-90°C
- RMN $^{31}P$ (81,015 MHz, $CDCl_3$) δ : + 27,7
- RMN $^1H$ (360 MHz, $CDCl_3$) δ : 1,35 (t, 6H, $OCH_2$-$\underline{CH_3}$, $3J_{HH}$ : 7Hz) ; 1,40 (d, 3H, $P(O)CH_3$,$3J_{PH}$ = 11Hz) ; 4,02 (q, 4H, $\underline{OCH_2}$-$CH_3$, $3J_{HH}$ = 7Hz).

**STADE B' : CHLORURE DE 5-PHENYLPENTANOYLE**

Dans un tricol de 500 $cm^3$ équipé d'un réfrigérant on introduit sous azote 51,44 g (289 mmol) d'acide 5-phénylpentanoïque et 44,65 g (375 mmol) de chlorure de thionyle fraîchement distillé. La suspension est agitée puis on porte progressivement le milieu à 60°C. Dès que cette température est atteinte le milieu est homogène, la température est maintenue 4h. Après être revenu à température ambiante, l'excès de chlorure de thionyle est chassé au rotavapor et le résidu est distillé sous pression réduite.
On obtient 54,5 g (277 mmol) de chlorure de 5-phénylpentanoyle, liquide incolore.
Rendement : 96 %

- Eb (1,33 Pa) = 118-120°C

- IR (film, $cm^{-1}$) : 1790 (νC = O)
- RMN $^1H$ ($CCl_4$) δ : 1,5 à 2 (m, 4H, $CH_2$) ; 2,4 à 3,1 (m, 4H, $CH_2C$=O et $CH_2Ph$) ; 7,2 (s, 5H, H aromatiques).

**STADE C' : 2-OXO-6-PHENYL-HEXYLPHOSPHONATE DE DIETHYLE**

**Méthode : SAVIGNAC P. et al., Tetrahedron Letters, 1976, pp 2829-2832.**

Dans un tricol de 500 $cm^3$ sec, on introduit sous azote 78 $cm^3$ (110 mmol) de n-butyllithium 1,41 N en solution dans l'hexane (le n-butyllithium est dosé juste avant l'emploi), et, à -60°C, on ajoute 80 $cm^3$ de tétrahydrofurane (THF) anhydre. On additionne (à -60°C) goutte à goutte 15,2 g (100 mmol) de méthylphosphonate de diéthyle dans 20 $cm^3$ de THF anhydre. La solution laiteuse est agitée 15 min à cette température puis on ajoute 20,93 g (110 mmol) d'iodure de cuivre pulvérulent en 10 min. La suspension brune devient noir au fur et à mesure de la remontée en température, -30°C en 30 min. L'agitation est maintenue à - 30°C pendant 1h. On refroidit de nouveau à - 60°C et 21,6 g (110 mmol) de chlorure de 5-phénylpentanoyle en solution dans 20 $cm^3$ de THF anhydre sont ajoutés en 15 min, le milieu reste noir ou se décolore. Le mélange réactionnel est agité 16 h en laissant remonter progressivement la température. On hydrolyse par de l'eau, les sels de cuivre sont filtrés sur célite et lavés au dichlorométhane. Après décantation, la couche aqueuse est extraite une fois au dichlorométhane. Les couches organiques réunies sont lavées à l'eau distillée puis séchées sur sulfate de sodium. Après concentration au rotavapor on obtient 33,2 g d'une huile jaune qui est purifiée par chromatographie sur une colonne de 670 g de silice.
Elution avec de l'éther pur, puis avec de l'acétate d'éthyle.
On obtient 26,5 g (85 mmol) de 2-oxo-6-phényl hexylphosphonate de diéthyle pur.
Rendement : 85 %

- IR (film, $cm^{-1}$) : 1700 (νC = O) ; 1240 (νP = O) ; 1020 - 950 (νP-O)
- RMN $^{31}P$ ($CDCl_3$ ; 81,015 MHz) δ = + 20,64
- RMN $^1H$ (360 MHz, $CDCl_3$) δ : 1,2 (t ; 6H ; $CH_3$, $3J_{HH}$ = 7Hz)) ; 1,52 (m, 4H, $H_4$, $H_5$) ; 2,5 (m ; 4H ; $H_3$, $H_6$) ; 2,92 (d, 2H, $H_1$, $J_{PH}$ = 22,47 Hz) ; 4,03 (m, 4H, $H_{1'}$, $3J_{PH}$ = $3J_{HH}$ = 7Hz) ; 7,04 (m, 3H, H aromatiques) ; 7,14 (m, 2H, H aromatiques).
-

| Analyse centésimale : $C_{16}$ $H_{25}$ $O_4$ P M = 312, 349 | | | | |
|---|---|---|---|---|
| Calc. % :<br>Mesuré : | C | 61,52<br>61,33 | H | 8,06<br>8,16 |

**STADE D : 1,1-ETHYLENEDIOXY-3-((E)-3-OXO-7-PHENYL-HEPT-1-EN-1-YL) CYCLOHEXANE**

Méthode : TAKACS J.M. et al, Tetrahedron Letters, 1986, vol. 27, pp 1257-1260.

A) Formation de l'anion du β-cétophosphonate obtenu au stade C'
Dans un tricol de deux litres, on introduit sous azote 36,4 g (116,7 mmol) du β-cétophosphonate en solution dans 420 $cm^3$ de THF anhydre, puis, en plusieurs fois et en agitant, 5,6 g d'hydrure de sodium à 50 % en suspension dans l'huile (116,7 mmol). La réaction est immédiate, on observe une coloration jaune.
En parallèle :
B) Réduction de l'ester obtenu au stade C au DIBAL (hydrure de diisobutylaluminium = DIBAL)
Dans un tricol d'un litre on introduit sous azote 21 g (105 mmol) d'ester en solution dans 420 $cm^3$ de toluène anhydre, après avoir refroidi à - 80°C on additionne goutte à goutte, en 1 h, 105 $cm^3$ (105 mmol) d'une solution de DIBAL 1N dans le toluène. 30 min après la fin de l'addition, on suit la réaction par chromatographie phase vapeur.
Ti : 100°C, program. 3° C/min

| | | | |
|---|---|---|---|
| tr (temps de rétention) | aldéhyde<br>alcool<br>ester | 993s<br>1153s<br>1252s | 89,4 %<br>3,2 %<br>4,8 % |

Le tricol A est refroidi à - 35°C, puis on transvase à l'aide d'une canule l'aldéhyde provenant de la réduction de l'ester (-78°C) sur l'anion en 20 min. Le bain froid étant retiré, la solution est ramenée en 40 min à température ambiante, puis agitée pendant 16 h. Le milieu hydrolysé par 100 $cm^3$ d'une solution saturée de chlorure

de sodium est agité pendant 1 h 30 min.
Après filtration sur célite et lavage au dichlorométhane, les couches organiques réunies sont lavées par une solution saturée de chlorure de sodium et séchées sur sulfate de sodium.
Après concentration on obtient 36,2 g d'une huile jaune.

L'ester obtenu au stade C précédent et le produit attendu ayant un coefficient de migration (Rf) identique, la séparation de ces composés sera réalisée par élimination de l'ester initial et de l'aldéhyde par distillation (Eb (1,33 Pa) = 60-70°C) avec une colonne très courte. La chromatographie en phase gazeuse après distillation met en évidence l'intérêt de ce procédé (absence d'ester et d'aldéhyde intermédiaire).
Le résidu est chromatographié sur une colonne de 750 g de silice (cyclohexane 80/éther 20) et fournit : 29,5 g (90 mmol) de 1,1-éthylènedioxy-3-((E)-3-oxo-7-phényl-hept-1-én-1-yl)cyclohexane et 5,26 g (16,86 mmol) de β-cétophosphonate initial (16 %).
Rendement : 85,7 %

- IR (film, $cm^{-1}$) : 1690 (νC = O) ; 1630 (νC = C) ; 970 (νHC = CH trans)
- RMN $^1H(CCl_4)$ δ : 0,8 à 2(m, 12H, $H_5$', $H_6$', $CH_2$ cyclo) ; 2,1 à 2,85 (m, 5H, $H_3$, $H_{4'}$, $H_{7'}$) ; 3,87 (s, 4H, $OCH_2$ - $CH_2O$) ; 5,94 (d, 1H, $H_{2'}$, J $H_{1'}$ $H_{2'}$ = 17Hz) ; 6,72 (dd, 1H, $H_{1'}$, $J_{H1'}H_{2'}$ = 16Hz, $J_{H1'H3}$ = 6,1 Hz) ; 7,18 (m, 5H, H aromatiques)
-

| Analyse centésimale : $C_{21}$ $H_{30}$ $O_3$ M = 330, 472 | | | | |
|---|---|---|---|---|
| Calc. % :<br>Mesuré : | C | 76,33<br>76,63 | H | 9,15<br>9,32 |

**STADE E : 1,1-ETHYLENEDIOXY-3-((E)-3-HYDROXY-7-PHENYL-HEPT-1-EN-1-YL)CYCLOHEXANE**

**(selon GEMAL A.L. et al, J. am. Chem. Soc., 1981, vol. 103, p 5454)**

Dans un tricol de 1 litre, on introduit une solution de 40,05 g (122,1 mmol) du composé obtenu au stade D dans 400 $cm^3$ de méthanol distillé, 45,5 g (122,12 mmol) de chlorure de cérium heptahydraté. La température passe de 16 à 19°C, on agite pendant 35 min, le sel passe progressivement en solution. Le ballon est refroidi à 9° C et on additionne en 55 min par petites fractions (mousses abondantes) 4,65 g (123 mmol) de borohydrure de sodium. En fin d'addition de l'hydrure les parois sont rincées par 70 $cm^3$ de méthanol, puis on laisse agiter quatre heures en revenant progressivement à température ambiante. Après avoir refroidi à l'aide d'un bain d'eau glacée, on hydrolyse à l'aide d'une solution d'acide chlorydrique 2 N jusqu'à pH neutre. Puis on ajoute 100 $cm^3$ d'une solution saturée de chlorure de sodium. Après décantation la phase aqueuse est extraite à l'éther, les phases organiques réunies sont lavées par une solution saturée de chlorure de sodium et séchées sur sulfate de sodium. Après concentration, on obtient 40,12 g de 1,1-éthylènedioxy-3-((E)-3-hydroxy-7-phényl-hept-1-én-1-yl)cyclohexane. L'huile visqueuse obtenue très légèrement colorée sera utilisée sans purification dans l'étape suivante. Rendement en brut : 99,5 %

- IR (film, $cm^{-1}$) : 3440 (νOH) ; 1660 (νC = C) ; 960 (νHC = CH trans)
- RMN $^1H(CCl_4)$ δ : 0,75 à 1,9 (m, 12H,$CH_2$ cyclo, $H_{5'}$, $H_{6'}$) ; 2 à 2,86 (m, 5H, $H_3$, $H_{4'}$, $H_{7'}$) ; 3,87 (s, 4H, $OCH_2$ - $CH_2O$) ; 3,85 à 4(m, 1H, $H_{3'}$) ; 5,43 (m, 2H, $H_{1'}$, $H_{2'}$) ; 7,13 (m, 5H, H aromatiques)
-

| Analyse centésimale : $C_{21}$ $H_{30}$ $O_3$ M = 330, 472 | | | | |
|---|---|---|---|---|
| Calc. % :<br>Mesuré : | C | 76,33<br>76,33 | H | 9,15<br>9,32 |

**STADE E' : REDUCTION ENANTIOSELECTIVE DE LA CETONE DU STADE D**

D'après E.J. COREY, R.K. BAKSHI, Tetrahedron Letters, 1990, 31, 611.

Dans un tricol de 100 $cm^3$ on introduit sous azote une solution composée de 2,055 g (6,22 mmol) de la cétone obtenu au stade D en solution dans 20 $cm^3$ de toluène anhydre (cette solution est préalablement séchée sur tamis moléculaire 4 Å durant 24 h). Puis 0,185 g (0,627 mmol ; 0,1 équivalent) de S-(-)-2-méthyl-CBS-oxazaborolidine (Lancaster) sont ajoutés, le catalyseur se solubilise en 20 min. Le milieu réactionnel est refroidi à - 78° C et on additionne en 25 min une solution composée de 1,35 g (11,2 mmol ; 2 équivalents) de catécholborane (Aldrich) dilué dans 30 $cm^3$

de toluène anhydre. Dès les premières gouttes, le milieu réactionnel incolore devient jaune. Après avoir agité à - 78°C pendant 21 h la solution trouble laiteuse est hydrolysée par 35 $cm^3$ d'une solution saturée de chlorure de sodium et on ajoute 50 $cm^3$ de dichlorométhane. Après être revenu à température ambiante on maintient l'agitation pendant 30 minutes puis on décante, la phase aqueuse est extraite 3 fois par 50 $cm^3$ de dichlorométhane. Les phases organiques réunies sont lavées 6 fois par 10 $cm^3$ d'une solution saturée de carbonate de sodium (la couche aqueuse verte est pratiquement incolore au dernier lavage), puis 2 fois par 20 $cm^3$ d'une solution saturée de chlorure de sodium et séchées sur sulfate de sodium. Après concentration on obtient 2,415 g d'une huile rouge visqueuse qui est chromatographiée sur une colonne de 60 g de silice. L'éluant cyclohexane 80/éther 20 fournit 250 mg de cétone initiale (12 %), cyclohexane 75/éther 25 fournit 1,750 g (5,264 mmol) de l'alcool attendu.
Rendement en produit chromatographié : 85 %

Le même mode opératoire est utilisé pour obtenir l'alcool S à partir du R-(-)-2-méthyl-CBS-oxazaborolidine (Lancaster).

**STADE F : 3-((E)-3-HYDROXY-7-PHENYL-HEPT-1-EN-1-YL)CYCLOHEXANONE**

D'après HUET F. et al, Synthesis, 1978, 63.

Dans un ballon de 250 $cm^3$, on introduit 50 $cm^3$ de dichlorométhane distillé et sous agitation 30 g de silice et 1,5 g d'une solution à 15 % d'acide sulfurique. Après avoir refroidi à l'aide d'un bain d'eau glacée on ajoute une solution composée de 8,22 g (24,9 mmol) du composé obtenu au stade E brut dans 35 $cm^3$ de dichlorométhane. Après avoir agité pendant 16 heures à température ambiante, on ajoute 1,5 g d'hydrogénocarbonate de sodium, on agite pendant 3 heures.
Après filtration sur verre fritté, la silice est lavée au dichlorométhane, le filtrat est lavé par une solution saturée de chlorure de sodium puis séché sur sulfate de sodium. Après concentration, on obtient 6,42 g d'un liquide épais de couleur jaune qui est chromatographié sur une colonne de 150 g de silice.
On isole (éluant cyclohéxane 65/éther 35) 5,508 g (19,25 mmol) de 3-((E)-3-hydroxy-7-phénylhept-1-èn-1-yl)cyclohexanone pur.
Rendement : 77,5 %

- IR (film, $cm^{-1}$) : 3320 (νOH) ; 1690 (νC = O)
- RMN $^1H(CCl_4)$ δ : 0,8 à 2,8 (m, 17H, H cyclo, $H_{4'}$ à $H_{7'}$) ; 3,93 (m, 2H, $H_{3'}$, et hydroxyle) ; 5,46 (m, 2H, $H_{1'}$, $H_{2'}$) ; 7,1 (5H, H aromatiques)
-

| Analyse centésimale : $C_{19}H_{26}O_2$ M = 286, 418 | | | | |
|---|---|---|---|---|
| Calc. % :<br>Mesuré : | C | 79,67<br>79,43 | H | 9,14<br>9,15 |

**STADE G : 3-((E)-3-TERT-BUTYLDIMETHYLSILYLOXY-7-PHENYL-HEPT-1-EN-1-YL)CYCLOHEXANONE**

Dans un tricol de 500 $cm^3$, on introduit sous azote une solution composée de 23,37 g de 1,8-diazabicyclo[5.4.0]undec-7-ène (153,5 mmol) dans 160 $cm^3$ de dichlorométhane anhydre et 19,3 g (128 mmol) de chlorure de tert-butyldiméthylsilane. Après avoir refroidi à 0°C, on additionne goutte à goutte une solution de 30,5 g (106,64 mmol) du composé obtenu au stade précédent dans 100 $cm^3$ de dichlorométhane anhydre. Le milieu est agité 48 h à température ambiante puis hydrolysé par 50 $cm^3$ d'eau distillée. La phase aqueuse est extraite par de l'éther. Les phases organiques sont lavées par une solution d'acide chlorhydrique 1N, puis par une solution d'hydrogénocarbonate de sodium et par de l'eau distillée ; après avoir été séchées sur sulfate de sodium et concentrées on obtient 44,45 g du produit attendu qui est purifié par chromatographie sur une colonne de 1130 g de silice.
On isole (éluant cyclohexane 97/éther 3) 40,1 g (100,2 mmol) de 3-((E)-3-tert-butyldiméthylsilyloxy-7-phénylhept-1-én-1-yl)cyclohexanone pur.
Rendement : 94%

- IR (film, $cm^{-1}$) : 1710 (νC = O) ; 1250 (νSi - $CH_3$) ; 1065 - 1090 (ν SiO-C) ; 840-870 (νSi - $CH_3$)
- RMN $^1H$($CCl_4$ sans TMS) : 0,0 (s, 6H, diméthylsilyl) ; 0,9 (s, 9H, tert-butylsilyl) ; 0,8 à 2,8 (17H, H cyclo, $H_{4'}$ à $H_{7'}$) ; 3,9 (1H, $H_{3'}$) ; 5,46 (m, 2H, $H_{1'}$, $H_{2'}$); 7,1 (5H, H aromatiques)

-

| Analyse centésimale : $C_{25}H_{40}O_2Si$ M = 400, 679 | | | | |
|---|---|---|---|---|
| Calc. % :<br>Mesuré : | C | 74,94<br>75,09 | H | 10,06<br>10,27 |

**PREPARATION 2 : 3-((E)-3-TERT-BUTYLDIMETHYLSILYLOXY-UNDEC-1-EN-1-YL)CYCLOHEXANONE**

En procédant comme dans la préparation 1 mais en remplaçant au stade C' le chlorure de 5-phényl pentanoyle par le chlorure de nonanoyle, on obtient le composé du titre.
I.R. (film, $cm^{-1}$) : 1705(ν C=O) ; 1245(ν Si-$CH_3$); 960 (ν HC = CH trans) ; 825 (ν Si-O-C)

**PREPARATION 3 : 3-((E)-3-TERT-BUTYLDIMETHYLSILYLOXY-OCT-1-EN-1-YL)CYCLOHEXANONE**

selon Corey E.J. et al, J. Am. Chem. Soc. 1972, vol. 94, pp 7210-7211

**PREPARATIONS 4 A 12**

En procédant comme dans la préparation 1 mais en utilisant au stade C' l'halogénure d'acyle approprié, on obtient successivement :

**PREPARATION 4 : 3-((E)-3-TERT-BUTYLDIMETHYLSILYLOXY-6-PHENOXY-HEX-1-EN-1-YL)CYCLOHEXANONE**

**PREPARATION 5 : 3-((E)-3-TERT-BUTYLDIMETHYLSILYLOXY-13-PHENOXY-TRIDEC-1-EN-1-YL)CYCLOHEXANONE**

**PREPARATION 6 : 3-((E)-3-TERT-BUTYLDIMETHYLSILYLOXY-4-(4-CHLOROPHENYL)BUT-1-EN-1-YL)CYCLOHEXANONE**

**PREPARATION 7 : 3-((E)-3-TERT-BUTYLDIMETHYLSILYLOXY-6-(3,4-DIMETHOXYPHENYL)-HEX-1-EN-1-YL) CYCLOHEXANONE**

**PREPARATION 8 : 3-((E)3-TERT-BUTYLDIMETHYLSILYLOXY-4,4-DIMETHYL-PENT-1-EN-1-YL)CYCLOHEXANONE**

**PREPARATION 9 : 3-((E)-3-TERT-BUTYLDIMETHYLSILYLOXY-4-(4-TRIFLUOROMETHYLPHENYL)-BUT-1-EN-1-YL)CYCLOHEXANONE**

**PREPARATION 10 : 3-((E)-3-TERT-BUTYLDIMETHYLSILYLOXY-DEC-1-EN-1-YL)CYCLOHEXANONE**

**PREPARATION 11 : 3-((E)-3-TERTBUTYLDIMETHYLSILYLOXY-5-CYCLOPENTYL-PENT-1-EN-1-YL)CYCLOHEXANONE**

**PREPARATION 12 : 3-((E)-3-TERT-BUTYLDIMETHYLSILYLOXY-7-PHENYL-HEPT-1-EN-1-YL)TETRALONE**

En procédant comme dans la préparation 1 mais en remplaçant le 3-oxo-cyclohexane carboxylate de méthyle obtenu au stade B par la 1-tétralone-3-carboxylate de méthyle obtenu après estérification de l'acide 1-tétralone-3-carboxylique (Biochem. Biophys. Acta, 1987 ; vol. 916 (2) ; pp 205-212), on obtient le composé de la préparation 12.

**EXEMPLE 1 : 1-HYDROXY-3-((E)-3-TERT-BUTYLDIMETHYSILYLOLXY-7-PHENYL-HEPT-1-EN-1-YL)CYCLOHEXANE-1-ACETATE D'ETHYLE**

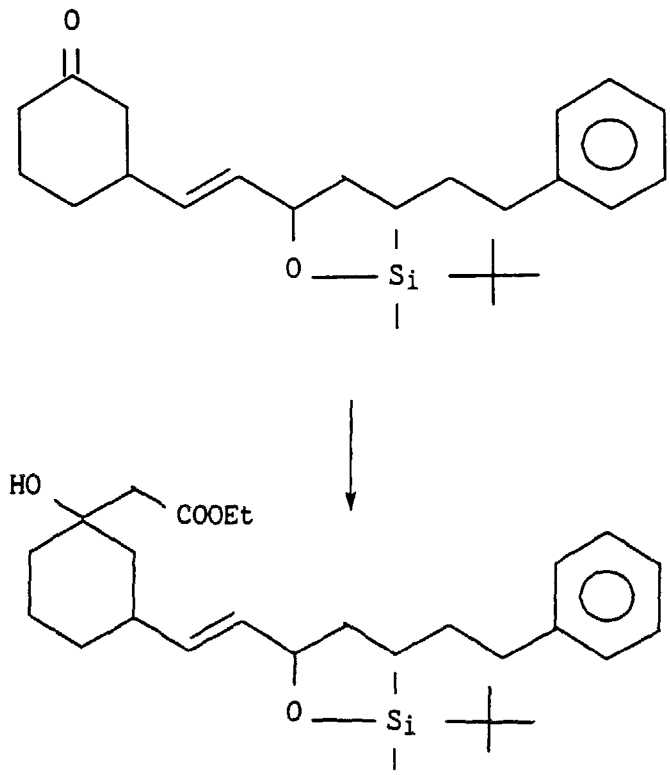

Dans un tricol de 1 litre sous azote, à une solution composée de 12,05 g (119 mmol) de diisopropylamine (fraîchement distillée) dans 400 $cm^3$ de THF anhydre on ajoute à - 80° C, goutte à goutte en trente minutes, 39,8 $cm^3$ (99,5 mmol) d'une solution de n-butyllithium (2,5 N dans l'hexane). Après 40 min d'agitation à - 78° C, on additionne goutte à goutte en 30 min 9,9 g (112,5 mmol) d'acétate d'éthyle (fraîchement distillé) en solution dans 70 $cm^3$ de THF anhydre. Le milieu est agité 30 min à -78° C puis on additionne goutte à goutte 24,84 g (62,1 mmol) du composé obtenu lors de la préparation 1 en solution dans 140 $cm^3$ de THF anhydre. Après avoir agité 16 h en laissant remonter la température, on hydrolyse par 70 $cm^3$ d'eau glacée.
La phase aqueuse est extraite à l'éther, les phases organiques réunies sont lavées deux fois par 50 $cm^3$ d'eau distillée et séchées sur sulfate de sodium.

**EXEMPLES 2 ET 3 : DIASTEREOISOMERES DU 1-HYDROXY-3-((E)-3-TERT-BUTYLDIMETHYLSILYLOXY-7-PHENYL-HEPT-1-EN-1-YL)CYCLOHEXANE-1-ACETATE D'ETHYLE**

**EXEMPLE 2 : (1S*,3S*)-1-HYDROXY-3-((E)-3-TERT-BUTYLDIMETHYLSILYLOXY-7-PHENYL-HEPT-1-EN-1-YL) CYCLOHEXANE-1-ACETATE D'ETHYLE**

**EXEMPLE 3 : (1R*,3S*)-1-HYDROXY-3-((E)-3-TERT-BUTYLDIMETHYLSILYLOXY-7-PHENYL-HEPT-1-EN-1-YL) CYCLOHEXANE-1-ACETATE D'ETHYLE**

32,95 g du composé obtenu à l'exemple 1 sont chromatographiés sur une colonne de 1200 g de silice pour obtenir une première séparation de deux isomères (Exemple 2 et Exemple 3) (éluant cyclohexane 92/ éther 8).
Remarque : la RMN du proton ($CCl_4$ sans TMS) permet de différencier (même à 60 MHz) les deux isomères en particulier au niveau des protons $CH_2COOEt$, ceux de l'exemple 2 ont un déplacement chimique à 2,33 et ceux de l'exemple 3 à 2,50. Ce qui à pour avantage de pouvoir suivre par RMN la séparation des deux isomères.
A ce niveau de la synthèse, il est impératif de disposer de produits extrêmement purs, c'est pourquoi les composés des exemples 2 et 3 isolés au cours de la chromatographie sont repris en Chromatographie Liquide Haute Performance ou CLHP (Colonne R SIL $SiO_2$ 10µ, L 250, Ø 22, éluant cyclohexane/éther (85/15), débit 8 $cm^3$/min, détecteur UV 260 nm, injection : 150 à 200 mg)
On isole après chromatographie CLHP :

**EXEMPLE 2 : (1S*,3S*)-1-HYDROXY-3-((E)-3-TERT-BUTYLDIMETHYLSILYLOXY-7-PHENYL-HEPT-1-EN-1-YL) CYCLOHEXANE-1-ACETATE D'ETHYLE**

isomère le moins polaire
12,9 g (26,4 mmol)
Rendement : 42,5 %
-RMN$^1$H($CDCl_3$ sans TMS) δ :
0,00(d ; 6H ; diméthylsilyloxy) ; 0,86 (m ; 9H ; tert-butylsilyloxy) ; 1,03 (m ; 2H ; $H_{2'}$) ; 1,26 ; 1,19 à 1,9 (m ; 15 H ; méthyle ester ; $H_{4'}$ à $H_{6'}$ ; $H_{4''}$ à $H_{6''}$) ; 2,15 à 2,7 (m ; 3H ; $H_{3'}$ et $H_{7''}$) ; 2,33 (s ; 2H ; $H_2$) ; 3,2 (m ; 1H, hydroxyle) ; 3,96 (m ; 1H ; $H_{3''}$) ; 4,2 (q ; 2H ; méthylène ester ; J = 7 Hz) ; 5,4 (m ; 2H ; $H_{1''}$, $H_{2''}$) ; 7,1 (m ; 5H ; H aromatiques).

**EXEMPLE 3 :(1R*,3S*)-1-HYDROXY-3-((E)-3-TERT-BUTYLDIMETHYSILYLOXY-7-PHENYL-HEPT-1-EN-1-YL)CYCLOHEXANE-1-ACETATE D'ETHYLE**

isomère le plus polaire
15 g (30,73 mmol)
Rendement : 49,5 %
-RMN$^1$H($CDCl_3$ sans TMS) δ :
0,00 (d ; 6H ; diméthylsilyloxy) ; 0,9 (m ; 9 H ; tert-butylsilyloxy) ; 1,03 (m ; 2H ; $H_{2'}$) ; 1,26 ; 1,2 à 1,9 (m ; 15H ; méthyle ester, $H_{4'}$ à $H_{6'}$ ; $R_{4''}$ à $H_{6''}$) ; 2,15 à 2,6 (m ; 3H ; $H_{3'}$ et $H_{7''}$) ; 2,50 (s ; 2H ; $H_2$) ; 3,5 (m ; 1H : hydroxyle) ; 3,95 (m ; 1H ; $H_{3''}$) ; 4,18 (q ; 2H ; méthylène ester ; J = 7Hz) ; 5,4 (m ; 2H ; $H_{1''}$, $H_{2''}$) ; 7,1 (m ; 5H ; H aromatiques).
Caractéristiques physiques communes aux composés des exemples 2 et 3 :

- IR (film, $cm^{-1}$) (Exemples 2 et 3) 3500 (νOH) ; 1710 (ν C = O) ; 1250 - (ν$SiCH_3$) ; 1065 - 1090 (νSi-O-C) ; 830 et 770 (νSi-$CH_3$)
-

| Analyse centésimale : $C_{29}$ $H_{48}$ $O_4$ Si M = 488, 78 | | | | |
|---|---|---|---|---|
| Calc. % : | C | 71,33 | H | 9,9 |
| Mesuré : | | 71,46 | | 10,16 |

**EXEMPLE 4 : (1S*,3S*)-1-HYDROXY-3-((E)-3-HYDROXY-7-PHENYL-HEPT-1-EN-1-YL)CYCLOHEXANE-1-ACETATE D'ETHYLE**

Dans un ballon de 1 litre, on introduit 14,3 g (29,3 mmol) de composé de l'exemple 2 en solution dans 310 $cm^3$ de THF distillé, après avoir refroidi à 0°C, on additionne 42 cm3 (42 mmol) d'acide chlorhydrique 1N. Après être revenu à température ambiante, le milieu est agité pendant 64 h puis on ajoute 6,9 g (84 mmol) d'hydrogénocarbonate de sodium. Le milieu est concentré au rotavapor puis repris par 500 $cm^3$ d'éther. La phase aqueuse est extraite à l'éther, les phases organiques réunies sont lavées deux fois par 50 $cm^3$ d'eau distillée, séchées sur sulfate de sodium. Après concentration on obtient 10,95 g d'une huile correspondant au composé du titre.

**EXEMPLES 5 ET 6 :**

**DIASTEREOISOMERES DU (1S*,3S*)-1-HYDROXY-3-((E)-3-HYDROXY-7-PHENYL-HEPT-1-EN-YL)CYCLOHEXANE-1-ACETATE D'ETHYLE**

**EXEMPLE 5 (1S*,3S*)-1-HYDROXY-3-((3S*R*, E)-3-HYDROXY-7-PHENYL-HEPT-1-EN-1-YL)CYCLOHEXANE-1-ACETATE D'ETHYLE**

**EXEMPLE 6 : (1S*,3S*)-1-HYDROXY-3-((3R*S*, E)-3-HYDROXY-7-PHENYL-HEPT-1-EN-1-YL)CYCLOHEXANE-1-ACETATE D'ETHYLE**

Le composé obtenu à l'exemple 4 est chromatographié sur une colonne de 600 g de silice afin de séparer les deux isomères.
L'éluant cyclohexane 60/éther 40 fournit 3,62 g d'un mélange (composé de l'exemple 5 / composé de l'exemple 6) qui contient le composé de l'exemple 5 surtout (10/90), puis 3,190 g d'un second mélange (environ 50/50), puis 3,6 g d'un mélange (environ 15/85), soit en tout 10,41 g (27,83 mmol)

Rendement (déprotection + chromatographie) : 95 %
Les deux dernières fractions des mélanges sont reprises séparément en chromatographie liquide haute performance (CLHP) : éluant cyclohexane/acétate d'éthyle, 85/15 et fournissent 1,964 g du composé pur de l'exemple 5, et 3,50 g du composé pur de l'exemple 6 (9,37 mmol)
Rendement composé de l'exemple 6 : 32 %

- IR (film, $cm^{-1}$) : 3410 (νOH) ; 1720 (νC=O) ; 1185 (νC-O-C) ; 960 (νHC = CH trans)
- RMN$^1$H(360 MHz, CDCL3) δ; - composé de l'exemple 5 : (isomère le moins polaire) :
  (1S*,3S*)-1 -hydroxy-3-((3S*R*, E)-3-hydroxy-7-phényl-hept-1-én-1-yl)cyclohexane-1-acétate d'éthyle.

0,97 (m, 2H, $H_{2'}$) ; 1,22 ; 1,13 à 1,75 (m, 15H, méthyle ester ; $H_{4'}$ à $H_{6'}$ ; $H_{4''}$ à $H_{6''}$) ; 2,37 (s, 2H, $H_2$) ; 2,43(m, 1H, $H_{3'}$) ; 2,55 (m, 2H, $H_{7''}$) ; 3,95 (q, 1H, $H_{3''}$, $JH_{3''}H_{4'}$ = $JH_{3''}H_{2''}$ = 6,4 Hz) ; 4,10 (q, 2H, méthylène ester, J = 7,2 Hz) ; 5,35 (dd, 1H, $H_{2''}$ ; $J_{H1''H2''}$ = 15,5 Hz, $J_{H2''H3''}$ = 6,1Hz) ; 5,46 (dd, 1H, $H_{1''}$ ; $J_{H1''H2''}$ = 15,5 Hz, $J_{H1''H3'}$ = 6,1 Hz) ; 7,15 (m, 5H, H aromatiques)
composé de l'exemple 6 : (isomère le plus polaire) :
(1S*,3S*)-1-hydroxy-3-((3R*S*, E)-3-hydroxy-7-phényl-hept-1-én-1-yl)cyclohexane-1-acétate d'éthyle.

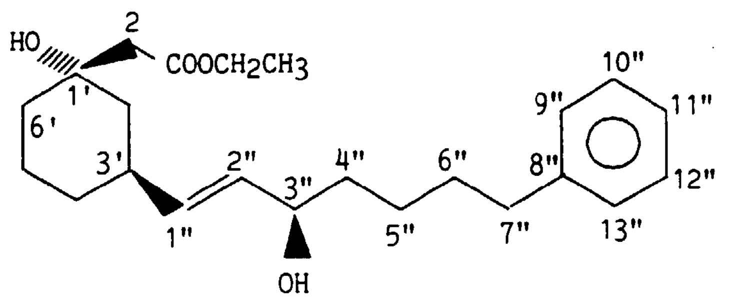

(exemple 6)

Caractéristiques spectrales : RMN$^{1H}$(360 MHz, $CDCL_3$) δ :
1,0 (m, 2H, $H_{2'}$) ; 1,26 ; 1,14 à 1,83 (m, 15H, méthyle ester ; $H_{4'}$ à $H_{6'}$ ; $H_{4''}$ à $H_{6''}$) ; 2,41 (s, 2H, $H_2$); 2,43 (m, 1H, $H_{3'}$) ; (t, 2H, $H_{7''}$) ; 4 (q, 1H, $H_{3''}$, $J_{H3''H4''}$ = $J_{H3''H2''}$ = 6,4 Hz) ; 4,16 (q, 2H, méthylène ester, J = 7,2 Hz) ; 5,40 (dd, 1H, $H_{2''}$ ; $J_{H1''H2''}$ = 15,5 Hz, $J_{H2''H3''}$ = 6,1Hz) ; 5,51 (dd, 1H,$H_{1''}$ ; $J_{H1''H2''}$ = 15,5 Hz, $J_{H1''H3'}$ = 6,1 Hz) ; 7,15 (m, 5H, H aromatiques).

- RMN$^{13}$C ($CDCl_3$, 90MHz) DEPT 135 et COSY (H, C). 172,8 ($C_1$) ; 142,6 ($C_{8''}$) ; 136,9 ($C_{1''}$) ; 131 ($C_{2''}$) ; 130,8 ($C_{10''}$ - $C_{12''}$) ; 128,4 ($C_{9''}$-$C_{13''}$) ; 125,6 ($C_{11''}$) ; 73,1 ($C_{3''}$) ; 69,8 ($C_{1'}$) ; 60,6 (C méthylène ester) ; 46,9 ($C_2$) ; 43,1 ($C_{2'}$) ; 37,2 ($C_{6'}$) ; 37 ($C_{5'}$) ; 36,6 ($C_{7''}$) ; 35,9 ($C_{3'}$) ; 32 ($C_{4'}$) ; 31,4 ($C_{6''}$) ; 25,1 ($C_{4''}$) ; 20,9 ($C_{5''}$) ; 14,2(C méthyl ester).
- UV sur le composé de l'exemple 6 (cyclohexane)
  λ max : 262 nm
  ε : 224
- Spectrographie de masse (IE, 20 eV) :
  composé de l'exemple 5
  m/z : 338 (M-$H_2O$)
  composé de l'exemple 6
-

| | | | |
|---|---|---|---|
| FAB+ : M Na+ | Calc. | 397,2354796 | |
| | Mesuré | 397,3367000 | Δ : 3,1 ppm |

Analyse centésimale : composé de l'exemple 5 : $C_{23}H_{34}O_4$ M = 374,52

| | | | | | | |
|---|---|---|---|---|---|---|
| Calc % | C | 73,76 | H | 9,15 | O | 17,09 |
| Mesuré | | 73,50 | | 9,22 | | 17,46 |

| | composé de l'exemple 6 | | |
|---|---|---|---|
| Mesuré | 73,50 | 9,21 | 17,55 |

**EXEMPLE 7 : ACIDE (1S*,3S*)-1-HYDROXY-3-((3R*S*, E)-3-HYDROXY-7-PHENYL-HEPT-1-EN-1-YL)CYCLOHEXANE-1-ACETIQUE**

Dans un ballon de 100 $cm^3$, on introduit 387 mg (1,035 mmol) du composé de l'exemple 6 en solution dans 20 $cm^3$ de méthanol, à la solution refroidie on ajoute 1,55 $cm^3$ d'hydroxyde de sodium 1N (1,55 mmol) et 20 $cm^3$ d'eau. On agite pendant 24 heures puis on concentre à l'évaporateur rotatif. Le produit brut est repris par 50 $cm^3$ d'eau et 50 $cm^3$ d'éther. La phase aqueuse est acidifiée par une solution d'acide chlorydrique 1N puis extraite 3 fois par 50 $cm^3$ d'éther. La couche étherée est séchée sur sulfate de sodium puis concentrée. Le produit brut est recristallisé (éther/cyclohexane) cristaux blancs.
Point de fusion = 87°C

- I.R (film, $cm^{-1}$) : 3640 et 2600 (νOH) ; 1730 (νC = O) ; 960 (νHC = CH trans)

- RMN (250 MHz, $CDCl_3$) δ :
1,0 (m, 2H, $H_{2'}$) ; 1,26 ; 1,15 à 1,9 (m, 12H, $H_{4'}$ à $H_{6'}$ ; $H_{4''}$ à $H_{6''}$) ; 2,47 (s, 2H, $H_2$) ; 2,43 (m, 1H, $H_{3'}$) ; 2,6 (t, 2H, $H_{7''}$, J = 7,4 Hz) ; 4,04 (q, 1H, $H_{3''}$, $J_{H_{3''}H_{4''}} = J_{H_{3''}H_{2''}}$ = 6,4 Hz) ; 4,4 (m, 3H, hydroxyles) ; 5,42 (dd, 1H, $H_{2''}$, $J_{H_{1''}H_{2''}}$ = 15,5 Hz , $J_{H_{2''}H_{3''}}$ = 6,1 Hz) ; 5,53 (dd, 1H,$H_{1''}$, $J_{H_{1''}H_{2''}}$ = 15,5 Hz, $J_{H_{1''}H_{3'}}$ = 6,1 Hz) ; 7,15 (m, 5H, H aromatiques)

-

| Analyse centésimale : $C_{21}$ $H_{30}$ $O_4$ M = 346,47 | | | | | | |
|---|---|---|---|---|---|---|
| Calc. % :<br>Mesuré : | C | 72,80<br>72,70 | H | 8,73<br>8,78 | O | 18,47<br>18,17 |

• ***Sodium(1S*,3S*)-1-hydroxy-3-((3R*S*,E)-3-hydroxy-7-phényl-hept-1-én-1-yl)cyclohexane-1-acétate:***

Dans un ballon de 250 $cm^3$, on introduit 501 mg (1,44 mmol) d'acide (1S*3S*)-1-hydroxy-3-((3R*S*, E)-3-hydroxy-7-phényl-hept-1-én-1-yl) cyclohexane-1-acétique que l'on met en solution dans 20 $cm^3$ de méthanol. Après avoir ajouté 10 $cm^3$ d'eau distillée, l'acide est neutralisé par une solution de soude 0,04 N en suivant la réaction au pH mètre (pH 8,3). La solution opalescente est concentrée à l'évaporateur rotatif pour éliminer l'alcool, puis lavée deux fois par 10 $cm^3$ d'éther distillé. La solution est de nouveau concentrée à l'évaporateur rotatif pour éliminer les traces d'éther, puis lyophilisée durant une nuit. Le solide blanc (526 mg) qui est collant est repris par 50 $cm^3$ d'eau distillée, la solution jaune claire est filtrée sur filtre millipore 0,45 μm avec pré-filtre. La solution limpide lyophilisée fournit 509 mg de sel blanc qui est tiré sous vide (6,65 Pa) pendant 10h à température ambiante.
$C_{21}H_{29}O_4Na$, M : 368
Solubilité du sel dans l'eau : 100 g/L.
Solubilité limpide légèrement coloré en jaune.
Stabilité de la solution : supérieure à 18 mois.

**EXEMPLE 8 : (1R*,3S*)-1-HYDROXY-3-((E)-3-HYDROXY-7-PHENYL-HEPT-1-EN-1-YL)CYCLOHEXANE-1-ACETATE D'ETHYLE**

En remplaçant dans l'exemple 4 le composé obtenu à l'exemple 2 par le composé de l'exemple 3, on obtient le produit du titre.

**EXEMPLES 9 ET 10 : DIASTEREOISOMERES DU (1R*,3S*)-1-HYDROXY-3-((E)-3-HYDROXY-7-PHENYL-HEPT-1-EN-1-YL)CYCLOHEXANE-1-ACETATE D'ETHYLE**

En procédant comme dans les exemples 5 et 6 mais en séparant par chromatographie les isomères du (1R*,3S*)-1-hydroxy-3-((E)-3-hydroxy-7-phényl-hept-1-èn-1-yl)cyclohexane-1-acétate d'éthyle, on obtient

**EXEMPLE 9 : (1R*,3S*)-1-HYDROXY-3-((3S*R*, E)-3-HYDROXY-7-PHENYL-HEPT-1-EN-1-YL)CYCLOHEXANE-1-ACETATE D'ETHYLE**

**EXEMPLE 10 : (1R*,3S*)-1-(HYDROXY)-3-((3R*S*, E)-3-HYDROXY-7-PHENYL-HEPT-1-EN-1-YL)CYCLOHEXANE-1-ACETATE D'ETHYLE**

Rendement : (pour les deux diastéréoisomères : 76 %)

- I.R (film, $cm^{-1}$) : 3410 (νOH) ; 1720 (νC = O) ; 1180 (νC-O-C) ; 960 (ν HC = CH trans)
- RMN (360 MHz, $CDCl_3$) δ :
  composé de l'exemple 9, le moins polaire :
  (1R*,3S*)-1-HYDROXY-3-((3S*R*, E)-3-HYDROXY-7-PHENYL-HEPT-1-EN-1-YL)CYCLOHEXANE-1-ACETATE D'ETHYLE
  0,97 (m, 2H, $H_{2'}$) ; 1,22 ; 1,13 à 1,9 (m, 15H, méthyle ester, ; $H_{4'}$ à $H_{6'}$ $H_{4''}$ à $H_{6''}$) ; 2 (m, 1H, $H_{3'}$) ; 2,52 (s, 2H, $H_2$) ; 2,53 (t, 2H, $H_{7''}$, J = 7,4 Hz) ; 3,95 (q, 1H, $H_{3''}$, $J_{H_{3''}H_{4''}} = J_{H_{3''}H_{2''}}$ = 6,4 Hz) ; 4,09 (m, 2H, méthylène ester) ; 5,37 (dd, 1H, $H_{2''}$, $J_{H_{1''}H_{2''}}$ = 15,5 Hz , $J_{H_{2''}H_{3''}}$ = 6,1 Hz) ; 5,5 (dd, 1H,$H_{1''}$, $J_{H_{1''}H_{2''}}$ = 15,5 Hz, $J_{H_{1''}H_{3'}}$ = 6,1 Hz) ; 7,15 (m, 5H, H aromatiques)
  composé de l'exemple 10, le plus polaire
  (1R*, 3S*)-1-HYDROXY-3-((3R *S*, E)-3-HYDROXY-7-PHENYL-HEPT-1-ENYL) CYCLOHEXANE-1-ACETATE D'ETHYLE
  0,97 (m, 2H, $H_{2'}$) ; 1,22 ; 1,13 à 1,8 (m, 15H, méthyle ester, ; $H_{4'}$ à $H_{6'}$ $H_{4''}$ à $H_{6''}$) ; 2 (m, 1H, $H_{3'}$) ; 2,5 (s, 2H, $H_2$) ; 2,53 (t, 2H, $H_{7''}$, J = 7,4 Hz) ; 3,96 (q, 1H, $H_{3''}$, $J_{H_{3''}H_{4''}} = J_{H_{3''}H_{2''}}$ = 6,4 Hz) ; 4,12 (m, 2H, méthylène ester) ; 5,37 (dd, 1H, $H_{2''}$, $J_{H_{1''}H_{2''}}$ = 15,5 Hz, $J_{H_{2''}H_{3''}}$ = 6,1 Hz) ; 5,5 (dd, 1H,$H_{1''}$, $J_{H1''H2''}$ = 15,5 Hz, $J_{H1''H3'}$ = 6,1 Hz) ; 7,15 (m, 5H, H aromatiques)
- SM (IE, 20 eV) :
  composés des exemples 9 et 10
  m/z : 356 (374 - $H_2O$)

**EXEMPLE 11 : 1-HYDROXY-3-((E)-3-TERT-BUTYLDIMETHYLSILYLOXY-UNDEC-1-EN-1-YL)CYCLOHEXANE-1-ACETATE D'ETHYLE**

En procédant comme dans l'exemple 1, mais en remplaçant le composé de la préparation 1 par le composé de la préparation 2, on obtient le composé du titre.

**EXEMPLES 12 ET 13 : DIASTEREOISOMERES DU 1-HYDROXY-3-((E)-3-TERT-BUTYLDIMETHYLSILYLOXY-UNDEC-1-EN-1-YL)CYCLOHEXANE-1-ACETATE D'ETHYLE**

En procédant comme dans les exemples 2 et 3 mais en séparant par chromatographie sur silice les isomères du composé de l'exemple 11, on obtient :

**EXEMPLE 12 : (1S*,3S*)-1-HYDROXY-3-((E)-3-TERT-BUTYLDIMETHYLSILYLOXY-UNDEC-1-EN-1-YL)CYCLOHEXANE-1-ACETATE D'ETHYLE**

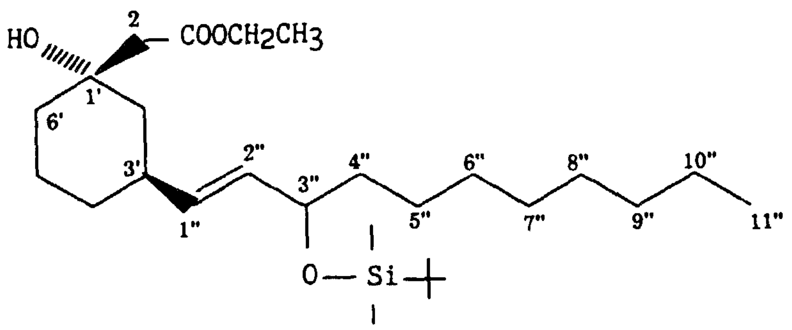

(Exemple 12)

**EXEMPLE 13 : (1R*,3S*)-1-HYDROXY-3-((E)-3-TERT-BUTYLDIMETHYLSILYLOXY-UNDEC-1-EN-1-YL)CYCLOHEXANE-1-ACETATE D'ETHYLE**

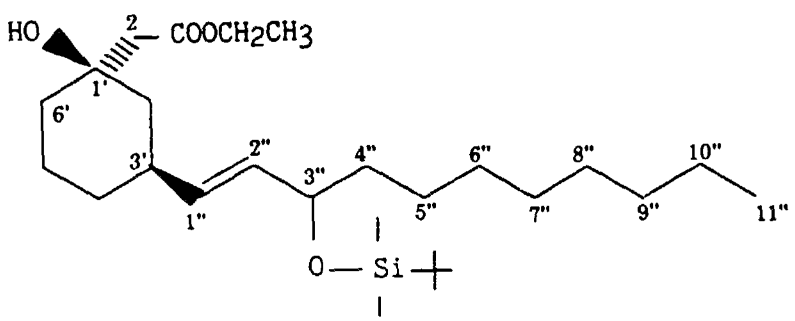

(Exemple 13)

Rendement global (les 2 isomères) : 86,5 %
Système chromatographique utilisé :

- CLHP Nucléosil 10 μ, éluant cyclohexane 90/éther 10
- IR (film, $cm^{-1}$) : (composés 12 et 13) : 3460 (νOH) ; 1710 (νC = O) ; 1245 (νSi-$CH_3$) ; 960 (νHC = CH trans) ; 825 - 765 (νSi-$CH_3$)
- RMN$^1$H ($CDCl_3$, 360 MHz) δ :
  composé de l'exemple 12 (le moins polaire)
  0,0 (d, 6H, diméthylsilyl) ; 0,84 (m, 12H, tert-butylsilyl, $H_{11''}$) ; 1,03 (m, 2H, $H_{2'}$) ; 1,26 ; 1,22 et 1,11 à 1,8 (m, 25 H, méthyl ester, $H_{4'}$ à $H_{6'}$, $H_{4''}$ à $H_{10''}$) ; 2,4 (s, 2H, $H_2$) ; 2,41 (m, 1H, $H_{3'}$) ; 3,37 (m, 1H, hydroxyle) ; 3,96 (q, 1H, $H_{3''}$, $J_{H_{3''}H_{4''}} = J_{H_{3''}H_{2''}}$ = 6,4 Hz) ; 4,16 (q, 2H, méthylène ester, J = 7,2 Hz) ; 5,31 (dd, 1H, $H_{2''}$, $J_{H_{1''}H_{2''}}$ = 15,5 Hz, $J_{H_{2''}H_{3''}}$ = 6,1Hz) ; 5,38 (dd, 1H, $H_{1''}$, $J_{H_{1''}H_{2''}}$ = 15,5 Hz, $J_{H_{1''}H_{3'}}$ = 6,1 Hz).
  composé de l'exemple 13 (le plus polaire)
  0,0 (d, 6H, diméthylsilyl) ; 0,85 (m, 12H, tert-butylsilyl, $H_{11''}$) ; 1,02 (m, 2H, $H_{2'}$) ; 1,26 ; 1,22 et 1,1 à 1,8 (m, 25 H, méthyl ester, $H_{4'}$ à $H_{6'}$, $H_{4''}$ à $H_{10''}$) ; 2,02 (m, 1H, $H_{3'}$) ; 2,57 (m, 2H, $H_2$) ; 3,83 (m, 1H, hydroxyle) ; 3,97 (q, 1H, $H_{3''}$, $J_{H_{3''}H_{4''}} = J_{H3''H2''}$ = 6,4 Hz) ; 4,17 (q, 2H, méthylène ester, J = 7,2 Hz) ; 5,32 (dd, 1H, $H_{2''}$, $J_{H_{1''}H_{2''}}$ = 15,5 Hz, $J_{H_{2''}H_{3''}}$ = 6,1Hz) ; 5,40 (dd, 1H, $H_{1''}$, $J_{H_{1''}H_{2''}}$ = 15,5 Hz, $J_{H_{1''}H_{3'}}$ = 6,1 Hz).

**EXEMPLE 14 : (1S*,3S*)-1-HYDROXY-3-((E)-3-HYDROXY-UNDEC-1-EN-1-YL)CYCLOHEXANE-1-ACETATE D'ETHYLE**

En procédant comme pour l'exemple 4 mais en remplaçant le composé de l'exemple 2 par le composé de l'exemple 12, on obtient le produit du titre.

**EXEMPLES 15 ET 16 :**

En procédant comme dans les exemples 5 et 6 mais en séparant les diastéréoisomères de l'exemple 14, on obtient :

**EXEMPLE 15 : (1S*,3S*)-1-HYDROXY-3-((3S*R*, E)-3-HYDROXY-UNDEC-1-EN-1-YL)CYCLOHEXANE-1-ACETATE D'ETHYLE**

**EXEMPLE 16 : (1S*,3S*)-1-HYDROXY-3-((3R*S*, E)-3-HYDROXY-UNDEC-1-EN-1-YL)CYCLOHEXANE-1-ACETATE D'ETHYLE**

Eluant : cyclohexane/acétate d'éthyle (85/15)

- I.R (film, $cm^{-1}$) : composés 15 et 16 : 3400 (νOH) ; 1710 (νC = O) ; 1200 (ν C-O-C)

- RMN$^1$H ($CDCl_3$, 360 MHz) δ :
  composé de l'exemple 15 (le moins polaire)
  0,85 (m, 3H, $H_{11''}$) ; 1,05 (m, 2H, $H_{2'}$) ; 1,25 ; 1,24 et 1,16 à 1,83 (m, 25H, méthyle ester, $H_{4'}$ à $H_{6'}$, $H_{4''}$ à $H_{10''}$) ; 2,40 (s, 2H, $H_2$) ; 2,42 (m, 1H, $H_{3'}$) ; 3,99 (q, 1H, $J_{H_{3''}H_{4''}} = J_{H_{3''}H_{2''}}$ = 6,4 Hz) ; 4,15 (q, 2H, méthylène ester, J = 7,2 Hz) ; 5,42 (dd, 1H, $H_{2''}$, $J_{H_{1''}H_{2''}}$ = 15,5 Hz, $J_{H_{2''}H_{3''}}$ = 6,1Hz) ; 5,52 (dd, 1H, $H_{1''}$, $J_{H_{1''}H_{2''}}$ , = 15,5 Hz, $J_{H_{1''}H_{3'}}$ = 6,1 Hz).
  composé de l'exemple 16 (le plus polaire)
  0,85 (t, 3H, $H_{11''}$) ; 1,05 (m, 2H, $H_{2'}$) ; 1,25 ; 1,24 et 1,16 à 1,83 (m, 25H, méthyle ester, $H_{4'}$ à $H_{6'}$, $H_{4''}$ à $H_{10''}$) ; 2,40 (s, 2H, $H_2$) ; 2,42 (m, 1H, $H_{3'}$) ; 4 (q, 1H, $H_{3''}$ $J_{H_{3''}H_{4''}} = J_{H_{3''}H_{2''}}$ = 6,4 Hz) ; 4,15 (q, 2H, méthyl ester, J = 7,2 Hz) ; 5,42 (dd, 1H, $H_{2''}$, $J_{H_{1''}H_{2''}}$ = 15,5 Hz, $J_{H_{2''}H_{3''}}$ = 6,1Hz) ; 5,52 (dd, 1H, $H_{1''}$, $J_{H_{1''}H_{2''}}$ = 15,5 Hz, $J_{H_{1''}H_{3'}}$ = 6,1 Hz).
  Composés des exemples 15 et 16 spectographie de masse (IE, 20 ev) : m/z : 318 (354-$2H_2O$)

**EXEMPLE 17 : (1R*,3S*)-1-HYDROXY-3-((E)-3-HYDROXY-UNDEC-1-EN-1-YL)CYCLOHEXANE-1-ACETATE D'ETHYLE**

En procédant comme pour l'exemple 4 mais en remplaçant le composé de l'exemple 2 par le composé de l'exemple 13 on obtient le produit du titre.

**EXEMPLES 18 ET 19 :**

En procédant comme pour les exemples 5 et 6 mais en séparant les diastéréoisomères de l'exemple 17, on obtient :

**EXEMPLE 18 : (1R*,3S*)-1-HYDROXY-3-((3S*R*, E)-3-HYDROXY-UNDEC-1-EN-1-YL)CYCLOHEXANE-1-ACETATE D'ETHYLE**

**EXEMPLE 19 : (1R*, 3S*)-1-HYDROXY-3-((3R*S*, E)-3-HYDROXY-UNDEC-1-EN-1-YL)CYCLOHEXANE-1-ACETATE D'ETHYLE**

- éluant : cyclohexane 80/ acétate d'éthyle 20
  Composé de l'exemple 18 (le moins polaire) :
  rendement : 37 %
  Composé de l'exemple 19 (le plus polaire) :
  Rendement : 42,5 %

- IR (film, $cm^{-1}$) composés 18 et 19 : 3400 (νOH) ; 1720 (νC = O) ; 1200 (ν C-O-C)

**EXEMPLES 20 A 25** :

En procédant comme dans les exemples 1 à 6 mais en partant dans l'exemple 1 du composé de la préparation 3 et en permettant le greffage en position 1 de la cyclohexanone du butyrate de méthyle au lieu de l'acétate d'éthyle, on obtient successivement :

**EXEMPLE 20 :1-HYDROXY-3-((E)-3-TERT-BUTYLDIMETHYLSILYLOXY-OCT-1-EN-1-YL)CYCLOHEXANE-1-BUTYRATE DE METHYLE**

**EXEMPLE 21 : (1S*,3S*)-1-HYDROXY-3-((E)-3-TERT-BUTYLDIMETHYLSILYLOXY-OCT-1-EN-1-YL)CYCLOHEXANE-1-BUTYRATE DE METHYLE**

**EXEMPLE 22 : (1R*,3S*)-1-HYDROXY-3-((E)-3-TERT-BUTYLDIMETHYLSILYLOXY-OCT-1-EN-1-YL)CYCLOHEXANE-1-BUTYRATE DE METHYLE**

**EXEMPLE 23 : (1S*,3S*)-1-HYDROXY-3-((E)-3-HYDROXY-OCT-1-EN-1-YL)CYCLOHEXANE-1-BUTYRATE DE METHYLE**

**EXEMPLE 24 : (1S*,3S*)-1-HYDROXY-3-((3S*R*, E)-3-HYDROXY-OCT-1-EN-1-YL)CYCLOHEXANE-1-BUTYRATE DE METHYLE**

**EXEMPLE 25 : (1S*,3S*)-1-HYDROXY-3-((3R*S*, E)-3-HYDROXY-OCT-1-EN-1-YL)CYCLOHEXANE-1-BUTYRATE DE METHYLE**

**EXEMPLES 26 A 28** :

En procédant comme dans les exemples 4 à 6, mais en partant du composé obtenu à l'exemple 22, on obtient :

**EXEMPLE 26 : (1R*,3S*)-1-HYDROXY-3-((E)-3-HYDROXY-OCT-1-EN-1-YL) CYCLOHEXANE-1-BUTYRATE DE METHYLE**

**EXEMPLE 27 : (1R*,3S*)-1-HYDROXY-3-((3S*R*, E)-3-HYDROXY-OCT-1-EN-1-YL) CYCLOHEXANE-1-BUTYRATE DE METHYLE**

**EXEMPLE 28 : (1R*,3S*)-1-HYDROXY-3-((3R*S*, E)-3-HYDROXY-OCT-1-EN-1-YL) CYCLOHEXANE-1-BUTYRATE DE METHYLE**

**EXEMPLES 29 A 32** :

En soumettant les composés obtenus aux exemples 24, 25, 27, et 28 à un traitement par l'hydroxyde de potassium, on obtient successivement :

**EXEMPLE 29 : (1S*,3S*)-1-HYDROXY-3-((3S*R*, E)-3-HYDROXY-OCT-1-EN-1-YL) CYCLOHEXANE-1-BUTYRATE DE POTASSIUM**

**EXEMPLE 30 : (1S*,3S*)-1-HYDROXY-3-((3R*S*, E)-3-HYDROXY-OCT-1-EN-1-YL) CYCLOHEXANE-1-BUTYRATE DE POTASSIUM**

**EXEMPLE 31 : (1R*,3S*)-1-HYDROXY-3-((3S*R*, E)-3-HYDROXY-OCT-1-EN-1-YL) CYCLOHEXAHE-1-BUTYRATE DE POTASSIUM**

**EXEMPLE 32 : (1R*,3S*)-1-HYDROXY-3-((3R*S*, E)-3-HYDROXY-OCT-1-EN-1-YL) CYCLOHEXANE-1-BUTYRATE DE POTASSIUM**

**EXEMPLES 33 A 38 :**

En procédant comme dans les exemples 1 à 6, mais en utilisant lors de l'exemple 1 le composé obtenu dans la préparation 3 au lieu du composé de la préparation 1, on obtient successivement :

**EXEMPLE 33 : 1-HYDROXY-3-((E)-3-TERT-BUTYLDIMETHYLSILYLOXY-OCT-1-EN-1-YL) CYCLOHEXANE-1-ACETATE D'ETHYLE**

**EXEMPLE 34 : (1S*,3S*)-1-HYDROXY-3-((E)-3-TERT-BUTYLDIMETHYLSILYLOXY-OCT-1-EN-1-YL) CYCLOHEXANE-1-ACETATE D'ETHYLE**

**EXEMPLE 35 : (1R*,3S*)-1-HYDROXY-3-((E)-3-TERT-BUTYLDIMETHYLSILYLOXY-OCT-1-EN-1-YL) CYCLOHEXANE-1-ACETATE D'ETHYLE**

**EXEMPLE 36 : (1S*,3S*)-1-HYDROXY-3-((E)-3-HYDROXY-OCT-1-EN-1-YL) CYCLOHEXANE-1-ACETATE D'ETHYLE**

**EXEMPLE 37 : (1S*,3S*)-1-HYDROXY-3-((3S*R*, E)-3-HYDROXY-OCT-1-EN-1-YL) CYCLOHEXANE-1-ACETATE D'ETHYLE**

**EXEMPLE 38 : (1S*,3S*)-1-HYDROXY-3-((3R*S*, E)-3-HYDROXY-OCT-1-EN-1-YL) CYCLOHEXANE-1-ACETATE D'ETHYLE**

**EXEMPLES 39 A 41** :

En procédant comme dans les exemples 4 à 6 mais en partant du composé obtenu à l'exemple 35, on obtient successivement :

**EXEMPLE 39 : (1R*,3S*)-1-HYDROXY-3-((E)-3-HYDROXY-OCT-1-EN-1-YL) CYCLOHEXANE-1-ACETATE D'ETHYLE**

**EXEMPLE 40 : (1R*,3S*)-1-HYDROXY-3-((3S*R*, E)-3-HYDROXY-OCT-1-EN-1-YL) CYCLOHEXANE-1-ACETATE D'ETHYLE**

**EXEMPLE 41 : (1R*,3S*)-1-HYDROXY-3-((3R*S*, E)-3-HYDROXY-OCT-1-EN-1-YL) CYCLOHEXANE-1-ACETATE D'ETHYLE**

**EXEMPLES 42 A 45** :

En procédant comme dans l'exemple 7 mais en remplaçant le composé de l'exemple 6 par le composé de l'exemple 37, 38, 40, puis 41, on obtient successivement :

**EXEMPLE 42 : ACIDE(1S*,3S*)-1-HYDROXY-3-((3S*R*, E)-3-HYDROXY-OCT-1-EN-1-YL)CYCLOHEXANE-1-ACETIQUE**

**EXEMPLE 43 : ACIDE(1S*,3S*)-1-HYDROXY-3-((3R*S*)-3-HYDROXY-OCT-1-EN-1-YL)CYCLOHEXANE-1-ACETIQUE**

**EXEMPLE 44 : ACIDE(1R*,3S*)-1-HYDROXY-3-((3S*R*)-3-HYDROXY-OCT-1-EN-1-YL)CYCLOHEXANE-1-ACETIQUE**

**EXEMPLE 45 : ACIDE(1R*,3S*)-1-HYDROXY-3-((3R*S*)-3-HYDROXY-OCT-1-EN-1-YL)CYCLOHEXANE-1-ACETIQUE**

**EXEMPLES 46 A 54** :

En procédant comme dans l'exemple 1 mais en partant des composés obtenus lors des préparations 4 à 12, on obtient successivement :

**EXEMPLE 46 : 1-HYDROXY-3-((E)-3-TERT-BUTYLDIMETHYLSILYLOXY-6-PHENOXY-HEX-1-EN-1-YL)CYCLOHEXANE-1-ACETATE D'ETHYLE**

**EXEMPLE 47 : 1-HYDROXY-3-((E)-3-TERT-BUTYLDIMETHYLSILYLOXY-13-PHENOXY-TRIDEC-1-EN-1-YL)CYCLOHEXANE-1-ACETATE D'ETHYLE**

**EXEMPLE 48 : 1-HYDROXY-3-((E)-3-TERT-BUTYLDIMETHYLSILYLOXY-4-(4-CHLOROPHENYL)-BUT-1-EN-1-YL)CYCLOHEXANE-1-ACETATE D'ETHYLE**

**EXEMPLE 49 : 1-HYDROXY-3-((E)-3-TERT-BUTYLDIMETHYLSILYLOXY-6-(3,4-DIMETHOXYPHENYL)-HEX-1-EN-1-YL)CYCLOHEXANE-1-ACETATE D'ETHYLE**

**EXEMPLE 50 : 1-HYDROXY-3-((E)-3-TERT-BUTYLDIMETHYLSILYLOXY-4,4-DIMETHYL-PENT-1-EN-1-YL)CYCLOHEXANE-1-ACETATE D'ETHYLE**

**EXEMPLE 51 : 1-HYDROXY-3-((E)-3-TERT-BUTYLDIMETHYLSILYLOXY-4-(4-TRIFLUOROMETHYLPHENYL)-BUT-1-EN-1-YL)CYCLOHEXANE-1-ACETATE D'ETHYLE**

**EXEMPLE 52 : 1-HYDROXY-3-((E)-3-TERT-BUTYLDIMETHYLSILYLOXY-DEC-1-EN-1-YL)CYCLOHEXANE-1-ACETATE D'ETHYLE**

**EXEMPLE 53 : 1-HYDROXY-3-((E)-3-TERT-BUTYLDIMETHYLSILYLOXY-5-CYCLOPENTYL-PENT-1-EN-1-YL) CYCLOHEXANE-1-ACETATE D'ETHYLE**

**EXEMPLE 54 : 1-HYDROXY-3-((E)-3-TERT-BUTYLDIMETHYLSILYLOXY-7-PHENYL-HEPT-1-EN-1-YL)-1,2,3,4-TETRAHYDRO-NAPHTALENE-1-ACETATE D'ETHYLE**

**EXEMPLES 55 A 63** :

En procédant comme dans l'exemple 4 mais en remplaçant le composé de l'exemple 2 par les composés des exemples 46 à 54, on obtient successivement :

**EXEMPLE 55 : 1-HYDROXY-3-((E)-3-HYDROXY-6-PHENOXY-HEX-1-EN-1-YL)CYCLOHEXANE-1-ACETATE D'ETHYLE**

**EXEMPLE 56 : 1-HYDROXY-3-((E)-3-HYDROXY-13-PHENOXY-TRIDEC-1-EN-1-YL)CYCLOHEXANE-1-ACETATE D'ETHYLE**

**EXEMPLE 57 : 1-HYDROXY-3-((E)-3-HYDROXY-4-(4-CHLOROPHENYL)-BUT-1-EN-1-YL)CYCLOHEXANE-1-ACETATE D'ETHYLE**

**EXEMPLE 58 : 1-HYDROXY-3-((E)-3-HYDROXY-6-(3,4-DIMETHOXYPHENYL)-HEX-1-EN-1-YL)CYCLOHEXANE-1-ACETATE D'ETHYLE**

**EXEMPLE 59 : 1-HYDROXY-3-((E)-3-HYDROXY-4,4-DIMETHYL-PENT-1-EN-1-YL)CYCLOHEXANE-1-ACETATE D'ETHYLE**

**EXEMPLE 60 : 1-HYDROXY-3-((E)-3-HYDROXY-4-(4-TRIFLUOROMETHYLPHENYL)-BUT-1-EN-1-YL)CYCLOHEXANE-1-ACETATE D'ETHYLE**

**EXEMPLE 61 : 1-HYDROXY-3-((E)-3-HYDROXY-DEC-1-EN-1-YL)CYCLOHEXANE-1-ACETATE D'ETHYLE**

**EXEMPLE 62 : 1-HYDROXY-3-((E)-3-HYDROXY-5-CYCLOPENTYL-PENT-1-EN-1-YL)CYCLOHEXANE-1-ACETATE D'ETHYLE**

**EXEMPLE 63 : 1-HYDROXY-3-((E)-3-HYDROXY-7-PHENYL-HEPT- 1-EN-1-YL)-1,2,3,4-TETRAHYDRONAPHTALENE-1-ACETATE D'ETHYLE**

**EXEMPLES 64 ET 65 :**

En procédant comme dans les exemples 54 et 63 mais en remplaçant le 1,2,3,4-tetrahydro-naphtalène par le perhydro-naphtalène, on obtient successivement.

**EXEMPLE 64 : 1-HYDROXY-3-((E)-3-TERT-BUTYLDIMETHYLSILYLOXY-7-PHENYL-HEPT-1-EN-1-YL)-PERHYDRO-NAPHTALENE-1-ACETATE D'ETHYLE**

**EXEMPLE 65 : 1-HYDROXY-3-((E)-3-HYDROXY-7-PHENYL-HEPT-1-EN-1-YL)-PERHYDRO-NAPHTALENE-1-ACETATE D'ETHYLE**

**EXEMPLES 66 ET 67 :**

**EXEMPLE 66 : (1S*, 3S*)-1-HYDROXY-3-((E)-3-TERT-BUTYLDIMETHYLSILYLOXY-7-PHENYLHEPT-1-EN-1-YL) CYCLOHEXANE-1-N,N-DIMETHYL ACETAMIDE**

**EXEMPLE 67 : (1R*, 3S*)-1-HYDROXY-3-((E)-3-TERT-BUTYLDIMETHYLSILYLOXY-7-PHENYLHEPT-1-EN-1-YL) CYCLOHEXANE-1-N,N-DIMETHYL ACETAMIDE**

D'après T. CUVIGNY, P. HULLOT, M. LARCHEVEQUE et H. NORMANT, Compte rendus, 279, série C, 1974, 569.

A une solution composée de 0,631 g (6,24 mmol) de diisopropylamine (fraîchement distillée) dans 20 $cm^3$ de THF anhydre on ajoute à - 80° C goutte à goutte en dix min 3,8 $cm^3$ (5,19 mmol) d'une solution de n-butyllithium (1,36N dans l'hexane). Après quarante minutes d'agitation à - 78° C on additionne à - 60° C goutte à goutte en trente minutes 0,511 g (5,87 mmol) de N,N-diméthyl acétamide (fraîchement distillé) en solution dans 3 $cm^3$ de THF anhydre. Le milieu est agité trente minutes à - 60° C puis on additionne goutte à goutte 1,3 g (3,24 mmol) de cétone obtenue lors de la préparation 1 en solution dans 3 $cm^3$ de THF anhydre. Après avoir agité 16h en laissant remonter la température on hydrolyse par 20 $cm^3$ d'eau glacée.

La phase aqueuse est extraite au dichlorométhane, les phases organiques réunies sont lavées deux fois par 20 $cm^3$ d'une solution saturée de chlorure de sodium et séchées sur sulfate de sodium.

Après concentration on obtient 1,7 g de produit brut qui est chromatographié sur une colonne de 50 g de silice pour

obtenir une première séparation de deux isomères des exemples 66 et 67 (éluant cyclohexane / éther (50 / 50)).

**REMARQUE** :

La RMN du proton ($CCl_4$ sans TMS) permet de différencier (même à 100 MHz) les deux isomères en particulier au niveau des protons $\underline{CH_2}CONMe_2$, ceux du composé de l'exemple 66 ont un déplacement chimiqe à 2,33 et ceux du composé de l'exemple 67 à 2,49. Ce qui a pour avantage de pouvoir suivre par RMN la séparation des deux isomères.
A ce niveau de la synthèse il est impératif de disposer de produits extrêmement purs, c'est pourquoi les composés des exemples 66 et 67 isolés au cours de la chromatographie (cyclohexane/éther 40/60) sont repris en CLHP.
Colonne R SIL $SiO_2$ 10 µ, L 250, Ø 22
éluant : dichlorométhane / méthanol (99,1 / 0,9), débit 12 $cm^3$/min.
détecteur UV 260 nm.
On isole après chromatographie CLHP :

- (1S*, 3S*)-1-hydroxy-3-((E)-3-tert-butyldiméthylsilyloxy-7-phénylhept-1-en-1-yl)cyclohexane-1-N,N-diméthylacétamide(0,715 g (1,467 mmol) - rendement : 45,8 %),

- (1R*, 3S*)-1-hydroxy-3-((E)-3-tert-butyldiméthylsilyloxy-7-phénylhept-1-en-1-yl)cyclohexane-1-N,N-diméthyl acétamide(0,401 g (0,8228 mmol) rendement : 25,9 %)
  Rendement global : 71,7 %

- IR (film, $cm^{-1}$) composés des exemples 66 et 67 :
  3400 (ν OH) ; 1620 (ν C = O) ; 1250 (ν $Si\text{-}CH_3$) ; 1065-1090 (ν Si-O-C) ; 830 et 770 (ν $Si\text{-}CH_3$) ;
  RMN$^1$H ($CDCl_3$ sans TMS, 360 MHz)δ :
  composé de l'exemple 66 : (isomère le moins polaire)
  0,01(m ; 6H ; diméthylsilyloxy) ; 0,85 (m ; 9H ; tert-butylsilyloxy) ; 0,93 (m ; 2H ; $H_{2'}$) ; 1 à 1,87 (m ; 12 H ; $H_{4'}$ à $H_{6'}$ ; $H_{4''}$ à $H_{6''}$) ; 2,35 (s ; 2H ; $H_2$) ; 2,45 (m ; 1H ; $H_{3'}$) ; 2,55 (m ; 2H ; $H_{7''}$) ; 2,94 (s, 3H, N-$CH_3$) ; 3,02 (s, 3H, N-$CH_3$) ; 3,96 (m ; 1H ; $H_{3''}$) ; 5,1 (d ; 1H, hydroxyle) ; 5,36 (m ; 2H ; $H_{1''}$, $H_{2''}$) ; 7,2 (m ; 5H ; H aromatiques).
  composé de l'exemple 67 : (isomère le plus polaire)
  0,01(m ; 6H ; diméthylsilyloxy) ; 0,85(m ; 9H ; tert-butylsilyloxy) ; 1,01(m ; 1H ; $H_{4'}$ A) ; 1,27 (m ; $H_{2'}$A) ; 1,29 (m ; $H_{5''}$ et $H_{5'}$A) ; 1,37 (m ; $H_{6'}$A) ; 1,43 (m ; $H_{4''}$) ; 1,58 (t ; 2H ; $^3J$ = 7,5 ; $H_{6''}$) ; 1,65 (m ; $H_{4'}$B) 1,70 (m ; $H_{5'}$B) ; 1,84 (m ; 2H ; $H_{6'}$B et $H_{2'}$B) 1,96 (m ; 1H ; $H_{3'}$) ; 2,51 (m ; 2H ; $H_2$) ; 2,57 (t ; 2H ; 3J : 7,2 ; $H_{7''}$) ; 2,94 (s ; 3H ; N-$CH_3$) ; 3,02 (s ; 3H ; N-$CH_3$) ; 3,97 (m ; 1H ; $H_{3''}$) ; 5,32 (dd ; 1H ; $J_{2'', 3''}$ = 6,1 ; $J_{2'', 1''}$ = 15,4 ; $H_{2''}$) ; 5,40(dd ; 1H ; $J_{1'', 2''}$ = 15,4 ; $J_{1'', 3'}$ = 6,1 ; $H_{1''}$) ; 5,69 (d ; 1H ; 3J = 5,0 ; OH) ; 7,14 (m ; 3H ; $H_{9''}$, $H_{11''}$, $H_{13''}$) ; 7,24 (t ; 2H ; 3J = 7,5 ; $H_{10''}$, $H_{12''}$).
  Corrélation $^1H/^1H$ ($CDCl_3$) : ($H_{9''}$ ; $H_{10''}$) ; ($H_{9''}$ ; $H_{7''}$) ; (OH ; $H_2$) ; ($H_{1''}$ ; $H_{3'}$) ($H_{2''}$ ; $H_{3''}$) ; ($H_{3''}$ ; $H_{4''}$) ; ($H_{7''}$ ; $H_{6''}$) ; ($H_{3'}$ ; $H_{2'B}$) ; ($H_{3'}$ ; $H_{2'A}$) ; ($H_{3'}$ ; $H_{4'A}$) ; ($H_{3'}$ ; $H_{4'B}$) ($H_{6'B}$ ; $H_{6'A}$) ; ($H_{6'B}$ ; $H_{5'B}$) ; ($H_{6'B}$ ; $H_{5'A}$) ; ($H_{5'B}$ ; $H_{5'A}$) ; ($H_{5'B}$ ; $H_{4'A}$) ; ($H_{5'B}$ ; $H_{4'B}$) ; ($H_{4'A}$ ; $H_{4'B}$) ; ($H_{2'A}$ ; $H_{2'B}$) ; ($CH_3$-t.Bu ; Si-$CH_3$).

-

| Analyse centésimale $C_{29}H_{49}NO_3Si$ M : 487,348 | | | | | | |
|---|---|---|---|---|---|---|
| Calc % | C | 71,41 | H | 10,13 | N | 2,87 |
| Mesuré | | 71,48 | | 9,90 | | 3,02 |

**EXEMPLES 68 ET 69 :**

**EXEMPLE 68 : (1S*, 3S*)-1-HYDROXY-3-((3 S*R*, E)-3-HYDROXY-7-PHENYLHEPT-1-EN-1-YL)CYCLOHEXANE-1-N,N-DIMETHYL ACETAMIDE**

**EXEMPLE 69 : (1S*, 3S*)-1-HYDROXY-3-((3 R*S*, E)-3-HYDROXY-7-PHENYLHEPT-1-EN-1-YL)CYCLOHEXANE-1-N,N-DIMETHYL ACETAMIDE**

Dans un ballon de 25 $cm^3$ on introduit 0,626 g (1,28 mmol) de composé de l'exemple 66 en solution dans 14 $cm^3$ de THF distillé, après avoir refroidi 0°C on additionne 1,93 $cm^3$ (1,93 mmol) d'acide chlorhydrique 1N. Après être revenu à température ambiante le milieu est agité pendant 64 h puis on ajoute 0,336 g (4 mmol) d'hydrogénocarbonate

de sodium. Le milieu est concentré au rotavapor puis repris par 50 $cm^3$ d'éther. La phase aqueuse est extraite à l'éther, les phases organiques réunies sont lavées deux fois par 5 $cm^3$ d'eau distillée, séchées sur sulfate de sodium. Après concentration on obtient 0,650 g d'une huile qui est chromatographiée sur une colonne de 37 g de silice afin d'isoler les deux isomères correspondant aux exemples 68 et 69.
L'élution à l'acétate d'éthyle fournit 0,430 g d'un mélange des deux isomères (1,154 mmol)
Rendement (déprotection + chromatographie) : 90 %
Les isomères sont purifiés (ou séparés) en CLHP : éluant acétate d'éthyle et fournissent 0,405 g des composés des exemples 68 et 69 purs (1,087 mmol)
Rendement (déprotection + chromatographie + HPLC) : 85 %

- IR (film, $cm^{-1}$) :
  3410 (ν OH) ; 1620 (ν C = O) ; 1185 (ν C-O-C) ; 960 (ν HC=CH trans).

- RMN$^1$H ($CDCl_3$, 360 MHz)δ :
  composé de l'exemple 68 : (isomère le moins polaire)
  (1S*, 3S*)-1-hydroxy-3-((3 S*R*, E)-3-hydroxy-7-phénylhept-1-èn-yl)cyclohexane-1-N,N-diméthyl acétamide
  0,97(m, 2H, $H_{2'}$) ; 1,13 à 1,75 (m, 12H, $H_{4'}$ à $H_{6'}$ ; $H_{4''}$ à $H_{6''}$) ; 2,37 (s, 2H, $H_2$) ; 2,46 (m, 1H, $H_{3'}$) ; 2,55 (t, 2H, $H_{7''}$) ; 2,94 (s, 3H, N-$CH_3$) ; 3,02 (s, 3H, N-$CH_3$) ; 3,95 (q, 1H, $H_{3''}$, $J_{H3''H4''}$ = $J_{H3''H2''}$ = 6,4) ; 5,08(s, 1H, hydroxyle) ; 5,35 (dd, 1H, $H_{2''}$ ;$J_{H1''H2''}$ = 15,5, $J_{H2''H3''}$ = 6,1) ; 5,46 (dd, 1H, $H_{1''}$ ; $J_{H1''H2''}$ = 15,5, $J_{H1''H3''}$ = 6,1) ; 7,14(m ; 3H ; $H_{9''}$, $H_{11''}$, $H_{13''}$) ; 7,24 (t ; 2H ; $^3J$ = 7,5 ; $H_{10''}$, $H_{12''}$).
  composé de l'exemple 69 : (isomère le plus polaire)
  (1S*, 3S*)-1-hydroxy-3-((3 R*S*, E)-3-hydroxy-7-phénylhept-1-èn-yl)cyclohexane-1-N,N-diméthyl acétamide
  1,0(m, 2H, $H_{2'}$) ; 1,14 à 1,83 (m, 12H, $H_{4'}$ à $H_{6'}$ ; $H_{4''}$ à $H_{6''}$) ; 2,41(s, 2H, $H_2$) ; 2,43 (m, 1H, $H_{3'}$) ; 2,57 (t, 2H, $H_{7''}$) ; 2,94 (s, 3H, N-$CH_3$) ; 3,02 (s, 3H, N-$CH_3$) ; 3,98 (q, 1H, $H_{3''}$, $J_{H3''H4''}$ = $J_{H3''H2''}$ = 6,4) 5,40 (dd, 1H, $H_{2''}$, $J_{H1''H2''}$ = 15,5, $J_{H2''H3''}$ = 6,1) ; 5,51 (dd, 1H,$H_{1''}$, $J_{H1''H2''}$= 15,5 Hz, $J_{H1''H3'}$ = 6,1 Hz) ; 5,70 (m, 1H, hydroxyle) ; 7,14 (m ; 3H ; $H_{9''}$, $H_{11''}$, $H_{13''}$) ; 7,24 (t ; 2H ; 3J = 7,5 ; $H_{10''}$, $H_{12''}$).

| Analyse centésimale $C_{23}H_{34}$ N $O_3$ M : 373,261 | | | | | | |
|---|---|---|---|---|---|---|
| Calc %<br>Mesuré | C | 73,94<br>74,02 | H | 9,45<br>9,65 | N | 3,75<br>3,71 |

**EXEMPLES 70 ET 71 :**

**EXEMPLE 70 : (1R*, 3S*)-1-HYDROXY-3-((3 S*R*, E)-3-HYDROXY-7-PHENYLHEPT-1-EN-1-YL)CYCLOHEXANE-1-N,N-DIMETHYL ACETAMIDE**

**EXEMPLE 71 : (1R*, 3S*)-1-HYDROXY-3-((3 R*S*, E)-3-HYDROXY-7-PHENYLHEPT-1-EN-1-YL)CYCLOHEXANE-1-N,N-DIMETHYL ACETAMIDE**

On opère, comme pour le composé de l'exemple 66, sur 355 mg (0,728 mmol) de composé de l'exemple 67. Les 230 mg d'huile obtenue après chromatographie sont purifiés en CLHP (éluant acétate d'éthyle). On isole 214 mg (0,305 mmol) de composés des exemple 70 et 71 purs.
Rendement global = 214 mg (0,574 mmol) = 79 %

- I.R. (film, $cm^{-1}$) :
  3410 (ν OH) ; 1720 (ν C = O) ; 1180 (ν C-O-C) ; 960 (ν HC=CH trans).

- RMN ($CDCl_3$, 360 MHz)δ :
  composé des exemples 70 et 71
  (1R*, 3S*)-1-hydroxy-3-((E)-3-hydroxy-7-phénylhept-1-èn-yl)cyclohexane-1-N,N-diméthyl acétamide
  0,97(m, 2H, $H_{2'}$) ; 1,13 à 1,9 (m, 12H, $H_{4'}$ à $H_{6'}$ ; $H_{4''}$ à $H_{6''}$) ; 2 (m, 1H, $H_{3'}$) ; 2,52(s, 2H, $H_2$) ; 2,53 (t, 2H, H7", J = 7,4) ; 2,94 (s, 3H, N-$CH_3$) ; 3,02 (s, 3H, N-$CH_3$) ; 3,95 (q, 1H, $H_{3''}$, $JH_{3''}H_{4''}$ = $JH_{3''}H_{2''}$ = 6,4) ; 5,37 (dd, 1H, $H_{2''}$ ; $J_{H1''H2''}$ = 15,5, $J_{H2''H3''}$ = 6,1) ; 5,5 (dd, 1H, H1", $J_{H1''H2''}$ = 15,5, $J_{H1''H3'}$ = 6,1) ; 5,7 (m, 1H, hydroxyle) 7,14 (m ; 3H ; $H_{9''}$, $H_{11''}$, $H_{13''}$) ; 7,24 (t ; 2H ; 3J = 7,5 ; $H_{10''}$, $H_{12''}$).

| Analyse centésimale $C_{23}H_{34}$ N $O_3$ M : 373,261 | | | | | | |
|---|---|---|---|---|---|---|
| Calc %<br>Mesuré | C | 73,94<br>74,02 | H | 9,45<br>9,48 | N | 3,75<br>3,70 |

**EXEMPLES 72 ET 73 :**

En procédant comme dans les exemples 66 et 67 mais en remplaçant le N, N-diméthylacétamide par le N-méthyl, N-phénylacétamide, on obtient les composés des exemples suivants :

**EXEMPLE 72 : (1S*, 3S*)-1-HYDROXY-3-((E)-3-HYDROXY-7-PHENYLHEPT-1-EN-1-YL)CYCLOHEXANE-1-N-METHYL N-PHENYL ACETAMIDE**

**EXEMPLE 73 : (1R*, 3S*)-1-HYDROXY-3-((E)-3-HYDROXY-7-PHENYLHEPT-1-EN-1-YL)CYCLOHEXANE-1-N-METHYL N-PHENYL ACETAMIDE**

**EXEMPLES 74 ET 75 :**

En procédant comme dans les exemples 66 et 67 mais en remplaçant le N, N-diméthylacétamide par le N, N-dipropylacétamide, on obtient les composés des exemples suivants :

**EXEMPLE 74 : (1S*, 3S*)-1-HYDROXY-3-((E)-3-HYDROXY-7-PHENYLHEPT-1-EN-1-YL)CYCLOHEXANE-1-N,N-DIPROPYLACETAMIDE**

**EXEMPLE 75 : (1R*, 3S*)-1-HYDROXY-3-((E)-3-HYDROXY-7-PHENYLHEPT-1-EN-1-YL)CYCLOHEXANE-1-N,N-DIPROPYLACETAMIDE**

**EXEMPLE 76 : (1S*, 3S*)-1-HYDROXY-3-(E)-3-OXO-7-PHENYLHEPT-1-EN-1-YL)CYCLOHEXANE-1-ACETATE D'ETHYLE**

D'après : D. TAUB, R.D. HOFFSOMMER, C.H. KUO, H.L. SLATES, Z.S. ZELANSKI, N.L. WENDLER, Tetrahedron, 1973, 1447.

Dans un ballon de 25 $cm^3$, on introduit 0,120 g (0,320 mmol) de composé de l'exemple 5 en solution dans 10 $cm^3$ de dichlorométhane. On ajoute 2 g de manganèse activé, le milieu est agité pendant 16 h à température ambiante. On filtre sur célite, rince au dichlorométhane et concentre au rotavapor. On obtient 0,105 g d'une huile qui est chromatographiée en HPLC (éluant cyclohexane 90/ acétate d'éthyle 10) ; on recueille ainsi 0,097 g (0,260 mmol) de cétone pure.
Rendement (oxydation + chromatographie HPLC) : 81 %

- IR (film, $cm^{-1}$) : 3410 ($\nu$ OH) ; 1720 ($\nu$ C = O) ; 1185 ($\nu$ C-O-C) ; 960 ($\nu$HC = CH trans).
- RMN$^1$H ($CDCl_3$, 100 MHz) $\delta$ : 0,75 à 2,15 (m, 15 H, $H_{2'}$, méthyle ester, $H_{4'}$ à $H_{6'}$ ; $H_{5''}$, $H_{6''}$) ; 2,2 à 2,80 (m, 7H, $H_2$, $H_{3'}$ $H_{4''}$, $H_{7''}$) ; 3,57 (s, 1H, OH) ; 4,15 (q, 2H, méthylène ester, J = 7,2) ; 5,94 (d, 1H, $H_{2''}$, $J_{H1''H2''}$ = 16 ; 6,72 (dd, 1H, $H_{1''}$, $J_{H1''H2''}$ = 16, $J_{H1''H3''}$ = 6,1) ; 7,18 (m, 5H, H aromatiques)
-

| Analyse centésimale : $C_{23}$ $H_{32}$ $O_4$ M = 374,52 | | | | |
|---|---|---|---|---|
| Calc. % :<br>Mesuré : | C | 74,15<br>74,05 | H | 8,66<br>8,74 |

Le composé ainsi obtenu peut également être réduit en utilisant une réduction du type de celle décrite au stade E' de la préparation 1.
Le composé de l'exemple 7 peut également être oxydé en utilisant le procédé décrit dans le présent exemple.

## EXEMPLES PHARMACOLOGIQUES :

### EXEMPLE A : ETUDE DE LIAISON DES COMPOSES DE L'INVENTION AUX RECEPTEURS DU $LTB_4$(Leucotriene $B_4$)

L'étude de l'affinité des composés de l'invention pour les récepteurs du $LTB_4$ est réalisée sur des leukocytes polymorphonucléaires (PMN) humains connus pour la présence sur leurs membranes de récepteurs du $LTB_4$ (J. Immunol. 1982, vol. 129, p 1600).

**PROTOCOLE :**

**1) Obtention des PMN humains**

Les PMN humains sont isolés du sang veineux hépariné de donneurs sains par sédimentation sur dextran $T_{500}$. Le surnageant est centrifugé sur Ficoll Paque® et les érythrocytes résiduels sont éliminés par une lyse hypotonique. Les PMN sont lavés avec une solution saline isotonique de Hank (HBSS) exempte de $Ca^{2+}$ et $Mg^{2+}$. La pureté cellulaire est vérifiée par la technique d'exclusion au bleu Trypan.

**2) Etude de liaison aux récepteurs $LTB_4$**

La liaison aux PMN est réalisée dans du HBSS exempt de $Ca^{2+}$ et de $Mg^{2+}$ contenant 5mM de tampon HEPES. Les PMN ($10^6$ cellules) sont incubées pendant 20 min à 4°C avec 1nM de $[^3H]$ $LTB_4$ en présence ou en absence de $LTB_4$ non radioactif ou des composés de l'invention à diverses concentrations.

**RESULTATS** :

L'incubation des PMN humains avec le $[^3H]$ $LTB_4$ (1 nM) en présence de doses croissantes de $LTB_4$ non marqué permet un déplacement dose-dépendant du $[^3H]$ $LTB_4$ de ses sites de liaisons spécifiques.

Les composés de l'invention se révèlent être de puissants ligands des récepteurs $LTB_4$ puisque ils permettent à des concentrations de l'ordre de $10^{-6}$ à $10^{-7}$ M 50 % d'inhibition de la liaison de $[^3H]$ $LTB_4$ ($IC_{50}$).

A titre d'exemple, le composé de l'exemple 7 permet une inhibition de 50 % ($IC_{50}$) de la liaison du $[^3H]$ $LTB_4$ à une concentration de $2.10^{-7}$ M. Les composés des exemples 69 et 71 permettent également d'obtenir une $IC_{50}$ de $5.10^{-7}$ M et $2.10^{-6}$ M respectivement.

### EXEMPLE B : ETUDE DE L'ACTIVITE DES COMPOSES DE L'INVENTION SUR LE CHIMIOTACTISME DES PMN HUMAINS IN VITRO

Le $LTB_4$ provoque une activation de la migration spontanée des PMN humains. La mesure de l'inhibition de cette migration permet donc de tester l'activité antagoniste des composés de l'invention vis-à-vis du $LTB_4$.

**PROTOCOLE** :

Des PMN incubés dans de l'HBSS sans $Ca^{2+}$ ni $Mg^{2+}$ sont marqués au $^{51}$chrome (1 $\mu$ Ci/$10^6$ cellules) pendant 1 h à 37°C puis lavés deux fois avec de l'HBSS.

Les PMN sont ensuite remis en suspension à une concentration de $10^7$ cellules/$cm^3$ dans du tampon de Hank additionné d'albumine bovine à 1 %. Le $LTB_4$ et les composés de l'invention en solution dans du tampon de Hank sont ajoutés dans la partie inférieure des chambres BOYDEN-KELLER, deux filtres de nitrate de cellulose (Millipore, 3 $\mu$m) sont placés sur chaque puits et la partie supérieure des chambres est remplie avec la suspension cellulaire (Methods Enzymol 1988, vol. 162, pp 59-64).

Les chambres sont incubées pendant 150 min à 37°C sous une atmosphère de 95 % air humidifié et 5 % $CO_2$. La radioactivité du filtre inférieur est mesurée par un spectromètre à scintillation (Beckman LS 3801).

**RESULTATS** :

Le $LTB_4$ (0,1 à 100 nM) provoque une activation de la migration spontanée des PMN humains à travers les filtres des chambres BOYDEN KELLER.

Les composés de l'invention se révèlent être de puissants antagonistes du $LTB_4$ puisqu'ils inhibent de façon dose-dépendante le chimiotactisme cellulaire induit par $LTB_4$.

A titre d'exemple, le composé de l'exemple 7, jusqu'à une concentration de 50 $\mu$M, permet d'inhiber totalement l'action du $LTB_4$ sur la migration cellulaire.

**EXEMPLE C : ETUDE DE L'ACTIVITE DES COMPOSES DE L'INVENTION SUR LA CONTRACTION DE BANDELETTES PARENCHYMATEUSES DE POUMON**

Le $LTB_4$ a un effet contracteur sur des bandelettes parenchymateuses de poumon de cobaye isolées. L'activité antagoniste des composés de l'invention sur cette contraction, induite par le $LTB_4$, est testée.

**PROTOCOLE** :

Des bandelettes parenchymateuses pulmonaires de cobaye DUNKIN HARTLEY sont découpées et placées dans des bains contenant une solution de TYRODE oxygénée par un courant gazeux (95 % $O_2$ / 5 % $CO_2$). Les bandelettes sont raccordées à un polygraph (GOULD Statham) avec une tension isométrique de 400 mg. Après 1 h d'équilibre, on ajoute 1 µM de mépyramine et 10 µM d'atropine à la solution de Tyrode et les composés de l'invention sont testés pour leurs activités antagonistes. Les réponses contractiles sont enregistrées pendant 5 min. Les activités antagonistes des composés de l'invention sont testées par l'addition de ces composés dans le bain 2 min avant la stimulation de la contraction par 30 nM de $LTB_4$.

**RESULTATS** :

Les composés de l'invention s'avèrent être de remarquables antagonistes du $LTB_4$ puisqu'ils antagonisent la contraction parenchymateuse pulmonaire induite par $LTB_4$.

Les concentrations des composés de l'invention inhibant 50 % de la contraction ($IC_{50}$) s'avèrent étonnament faibles. A titre d'exemple, l'$IC_{50}$ du composé de l'exemple 7 est de 40 nM.

**EXEMPLE D : ETUDE DE L'ACTIVITE DES COMPOSES DE L'INVENTION SUR UN MODELE INFLAMMATOIRE TOPIQUE (psoriasis)**

Le psoriasis est une maladie chronique de la peau présentant un mécanisme inflammatoire. Il a été montré que les cellules T jouent un rôle majeur dans le processus pathologique. Notamment, l'infiltration dermale des neutrophiles dans les lésions psoriatiques a été relié à des facteurs chimiotactiques tel que $LTB_4$.
Le présent test permet de tester l'activité anti-inflammatoire des composés de l'invention sur de telles liaisons dermales.

**PROTOCOLE :**

(Current Test Protocols-Pharmacology Service-Panlabs-June 92).
L'acide arachidonique (2 mg dans 20 µl d'un mélange acétone/pyridine/eau (7/2/1)) est appliqué en topique sur les surfaces antérieures et postérieures de l'oreille droite d'une souris 30 min après une application similaire du composé à tester ou du véhicule (20 µl). Le gonflement de l'oreille est mesurée par une jauge de type Dyer après 60 min en tant qu'index de l'inflammation.
Le pourcentage d'inhibition est calculé suivant la formule :Ic-It/Ic × 100, dans laquelle Ic et It représentent l'augmentation de l'épaisseur (en mm) de l'oreille chez la souris contrôle et chez la souris traitée, respectivement.

**RESULTATS** :

Il apparaît que les composés de l'invention sont de puissants anti-inflammatoires in topique.
Par exemple, le composé de l'exemple 7 permet une diminution de 36 % du volume de l'oreille à une dose de 20 mg/oreille.

**EXEMPLE E : ETUDE DE LA TOXICITE AIGUE**

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La $DL_{50}$ entraînant la mort de 50 % des animaux, a été évaluée.
La $DL_{50}$ des produits testés est supérieure à 1000 $mg.kg^{-1}$ pour la plupart des composés étudiés ce qui indique la faible toxicité des composés de l'invention.

## Revendications

**1.** Composés de formule (I) :

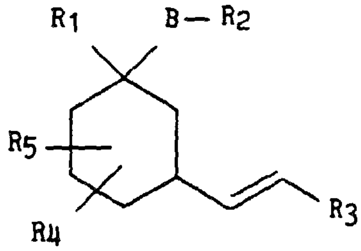

(I)

dans laquelle :

- B représente une chaine alkylène de 1 à 8 atomes de carbone non substituée ou substituée par 1 ou 2 radicaux alkyles,
- $R_1$ représente un atome d'hydrogène ou un radical choisi parmi hydroxy et alkoxy,
- $R_2$ représente un radical choisi parmi :

$$-CH_2-OH$$

et

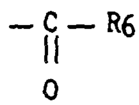

dans lequel $R_6$ représente un groupement choisi parmi hydroxyle, alkoxy, et

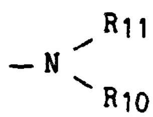

dans lequel $R_{10}$ et $R_{11}$ représentent, chacun indépendamment l'un de l'autre, un atome d'hydrogène, un radical alkyle, ou un groupement aryle ou arylalkyle, le groupement aryle ou arylalkyle étant non substitué ou substitué sur le noyau aryle par un ou plusieurs radicaux choisis parmi halogène, alkyle, alkoxy et trifluorométhyle, ou bien $R_{10}$ et $R_{11}$ forment ensemble avec l'atome d'azote qui les porte un hétérocycle choisi parmi pyrrolidine, pipéridine, pipérazine, morpholine, et thiomorpholine ;

- $R_3$ représente un groupement de formule :

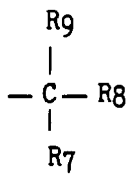

dans lequel :

- $R_7$ représente un radical choisi parmi hydroxy, alkoxy, et -O-$R_A$ dans lequel $R_A$ représente un acyle ou un groupement protecteur de la fonction hydroxyle,
- $R_9$ représente un atome d'hydrogène ou un radical choisi parmi alkyle, aryle, et arylalkyle, le groupement aryle ou arylalkyle étant non substitué ou substitué sur le noyau aryle par un ou plusieurs radicaux choisis parmi halogène, alkyle, alkoxy et trifluorométhyle,
- ou bien $R_7$ et $R_9$ forment un groupement oxo,

- et $R_8$ représente un groupement alkyle linéaire ou ramifié de 1 à 12 atomes de carbone non substitué ou substitué par un ou plusieurs radicaux choisis parmi :

  . hydroxyle,
  . halogène,
  . aryle non substitué,
  . aryle substitué par un ou plusieurs radicaux choisis parmi halogène, hydroxy, alkyle, alkoxy, et trifluorométhyle,
  . cycloalkyle non substitué,
  . cycloalkyle substitué par un ou plusieurs radicaux choisis parmi halogène, alkyle, oxo, et alkoxy,

- $R_8$ pouvant éventuellement contenir dans son squelette carboné :

  - un ou deux hétéroatomes choisis parmi azote, soufre, et oxygène,
  - une ou plusieurs liaisons insaturées,

- $R_4$ et $R_5$ représentent chacun indépendamment l'un de l'autre, un radical choisi parmi hydrogène et alkyle, ou bien, $R_4$ et $R_5$ forment ensemble avec le cyclohexane qui les porte un groupement 1,2,3,4-tetrahydronaphtalène ou un groupement perhydronaphtalène,
  étant entendu que lors de la description de la formule (I), et sauf précisions contraires :

- les termes "alkyle", "alkoxy" et "acyle" désignent des groupements linéaires ou ramifiés contenant de 1 à 6 atomes de carbone,

- le terme "aryle" désigne un groupement phényle ou naphtyle,

- le terme "liaison insaturée" désigne une double ou une triple liaison,

- le terme "cycloalkyle" désigne un groupement de 3 à 7 atomes de carbone,

leurs énantiomères et diastéréoisomères,
leurs isomères Z et E,
et leurs sels d'addition à un acide ou à une base pharmaceutiquement acceptable.

**2.** Composés selon la revendication 1 dans lesquels $R_8$ représente un groupement alkyle linéaire ou ramifié de 1 à 12 atomes de carbone non substitué,
leurs énantiomères et diastéréoisomères, leurs isomères Z et E, et leurs sels d'addition à un acide ou à une base pharmaceutiquement acceptable.

**3.** Composés selon la revendication 1 dans lesquels $R_8$ représente un groupement alkyle linéaire ou ramifié de 1 à 12 atomes de carbone substitué par un radical phényl non substitué ou substitué,
leurs énantiomères et diastéréoisomères, leurs isomères Z et E, et leurs sels d'addition à un acide ou à une base pharmaceutiquement acceptable.

**4.** Composés selon la revendication 1 dans lesquels $R_7$ représente un hydroxy,
leurs enantiomères et diastéréoisomères, leurs isomères Z et E, et leurs sels d'addition à un acide ou à une base pharmaceutiquement acceptable.

**5.** Composé selon la revendication 1 qui est le 1-hydroxy-3-((E)-3-tert-butyldiméthylsilyloxy-7-phényl-hept-1-èn-1-yl) cyclohexane-1-acétate d'éthyle et ses énantiomères et diastéréoisomères.

**6.** Composé selon la revendication 1 qui est le (1S*, 3S*)-1-hydroxy-3-((E)-3-tert-butyldiméthylsilyloxy-7-phenyl-hept-1-èn-1-yl)cyclohexane-1-acétate d'éthyle et ses énantiomères et diastéréoisomères.

**7.** Composé selon la revendication 1 qui est le (1S*, 3S*)-1-hydroxy-3-((3R*S*, E)-3-hydroxy-7-phényl-hept-1-èn-1-yl)cyclohexane-1-acétate d'éthyle et ses énantiomères et diastéréoisomères.

**8.** Procédé de préparation des composés de formule (I) caractérisé en ce que :

on fait réagir un composé de formule (II) :

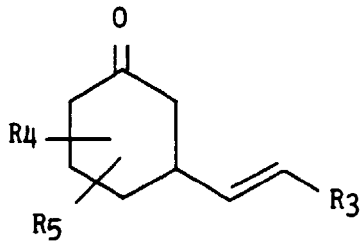

(II)

dans laquelle $R_3$, $R_4$, et $R_5$ sont tels que décrits dans la formule (I) avec un composé de formule (III) :

$$^{\ominus}B-R_2 \qquad (III)$$

dans laquelle $R_2$ et B sont tels que décrits dans la formule (I) pour obtenir le composé de formule ($I_a$) correspondant :

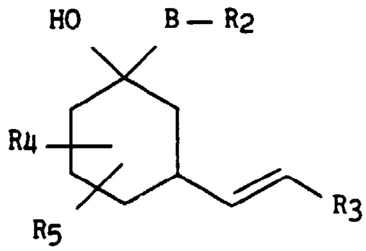

($I_a$)

dans laquelle $R_2$, $R_3$, $R_4$, $R_5$ et B sont tels que définis précédemment, qui peuvent être, si on le désire,

- soit soumis à l'action d'un composé de formule (IV) :

  $$R'^{\oplus} \qquad (IV)$$

  dans laquelle R' représente un alkyle pour obtenir les composés de formule ($I_b$) :

  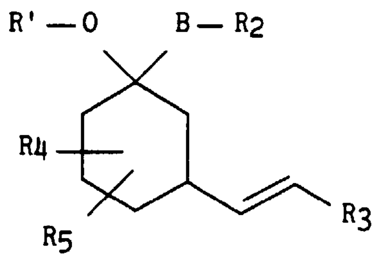

  ($I_b$)

  dans laquelle $R_2$, $R_3$, $R_4$, $R_5$, R' et B sont tels que définis précédemment,

- soit soumis au chlorure de tosyle puis à une réduction par l'hydrure d'aluminium et de lithium pour conduire au composé de formule ($I_c$) :

  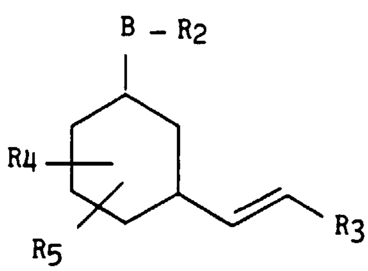

  ($I_c$)

  dans laquelle $R_2$, $R_3$, $R_4$, $R_5$ et B sont tels que définis précédemment,
  les composés de formule (I/a), (I/b), et (I/c) dans lesquels $R_9$ représente un atome d'hydrogène et $R_7$ représente un radical hydroxyle pouvant être oxydé afin d'obtenir les composés de formule (I/d) :

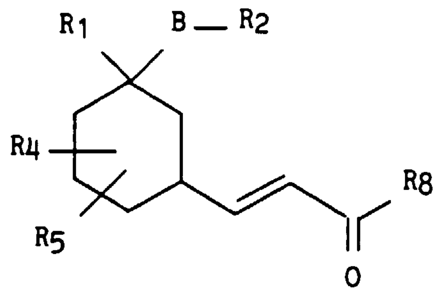

(I/d)

dans laquelle R1, R2, R4, R5, R8, et B sont tels que définis précédemment,

Les composés de formule (I/a), (I/b), (I/c), et (I/d) représentant l'ensemble des composés de formule (I), composés de formule (I) qui sont, si on le désire, séparés en leurs différents énantiomères ou diastéréoisomères, et salifiés le cas échéant avec un acide ou une base pharmaceutiquement acceptable.

**9.** Procédé selon la revendication 8 caractérisé en ce que la séparation des diastéréoisomères se réalise par chromatographie sur colonne.

**10.** Procédé selon la revendication 8 caractérisé en ce que la séparation des diastéréoisomères se réalise par chromatographie sur colonne et en ce que le groupement $R_7$ des composés de formule (I) selon la revendication 1 représente un groupement tert-butyldiméthylsilyloxy.

**11.** Composition pharmaceutique contenant comme principe actif au moins un composé selon la revendication 1 en combinaison avec un ou plusieurs excipients ou véhicules pharmaceutiquement acceptables.

**12.** Composition pharmaceutique selon la revendication 11 utile dans le traitement et la prévention des maladies inflammatoires et des conditions inflammatoires pathologiques.

**13.** Composition pharmaceutique selon la revendication 11 utile dans le traitement et la prévention de l'inflammation chronique ou aiguë, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux, de l'ischémie, de l'athérosclérose, de la détresse respiratoire, et du choc septique.

**Claims**

**1.** Compounds of formula (I):

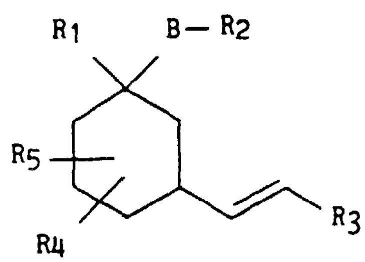

(I)

wherein:

- B represents an alkylene chain having from 1 to 8 carbon atoms that is unsubstituted or substituted by 1 or 2 alkyl radicals,
- $R_1$ represents a hydrogen atom or a radical selected from hydroxy and, alkoxy,
- $R_2$ represents a radical selected from:
  $-CH_2-OH$ and

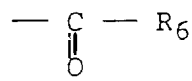

wherein $R_6$ represents a group selected from hydroxyl, alkoxy, and

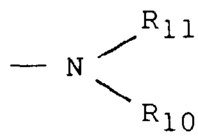

wherein $R_{10}$ and $R_{11}$ represent, each independently of the other, a hydrogen atom, an alkyl radical, or an aryl or arylalkyl group, the aryl or arylalkyl group being unsubstituted or substituted on the aryl ring by one or more radicals selected from halogen, alkyl, alkoxy and trifluoromethyl, or $R_{10}$ and $R_{11}$ form, together with the nitrogen atom carrying them, a heterocycle selected from pyrrolidine, piperidine, piperazine, morpholine and thiomorpholine;

- $R_3$ represents a group of the formula:

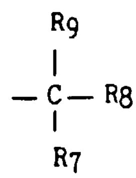

wherein:

- $R_7$ represents a radical selected from hydroxy, alkoxy, and -O-$R_A$ wherein $R_A$ represents an acyl radical or a protecting group for the hydroxyl function,
- $R_9$ represents a hydrogen atom or a radical selected from alkyl, aryl and arylalkyl, the aryl or arylalkyl group being unsubstituted or substituted on the aryl ring by one or more radicals selected from halogen, alkyl, alkoxy and trifluoromethyl,
- or $R_7$ and $R_9$ form an oxo group, and
- $R_8$ represents a linear or branched alkyl group having from 1 to 12 carbon atoms that is unsubstituted or substituted by one or more radicals selected from:
  . hydroxyl,
  . halogen,
  . unsubstituted aryl,
  . aryl that is substituted by one or more radicals selected from halogen, hydroxy, alkyl, alkoxy and trifluoromethyl,
  . unsubstituted cycloalkyl, and
  . cycloalkyl that is substituted by one or more radicals selected from halogen, alkyl, oxo and alkoxy,
- it being possible for $R_8$ optionally to contain in its carbon skeleton:
  - one or two hetero atoms selected from nitrogen, sulphur and oxygen,
  - one or more unsaturated bonds,
- $R_4$ and $R_5$ represent, each independently of the other, a radical selected from hydrogen and alkyl, or $R_4$ and $R_5$ form, together with the cyclohexane carrying them, a 1,2,3,4-tetrahydronaphthalene or a perhydronaphthalene group,
  it being understood that, unless otherwise specified, for the purpose of the description of formula (I):
- the terms "alkyl", "alkoxy" and "acyl" denote linear or branched groups having from 1 to 6 carbon atoms,

- the term "aryl" denotes a phenyl or naphthyl group,
- the term "unsaturated bond" denotes a double or triple bond,
- the term "cycloalkyl" denotes a group having from 3 to 7 carbon atoms,
their enantiomers and diastereoisomers,
their Z and E isomers, and
the addition salts thereof with a pharmaceutically acceptable acid or base.

**2.** Compounds according to claim 1 in which $R_8$ represents an unsubstituted linear or branched alkyl group having from 1 to 12 carbon atoms,
their enantiomers and diastereoisomers, their Z and E isomers, and the addition salts thereof with a pharmaceutically acceptable acid or base.

**3.** Compounds according to claim 1 in which $R_8$ represents a linear or branched alkyl group having from 1 to 12 carbon atoms that is substituted by an unsubstituted or substituted phenyl radical,
their enantiomers and diastereoisomers, their Z and E isomers, and the addition salts thereof with a pharmaceutically acceptable acid or base.

**4.** Compounds according to claim 1 in which $R_7$ represents hydroxy,
their enantiomers and diastereoisomers, their Z and E isomers, and the addition salts thereof with a pharmaceutically acceptable acid or base.

**5.** Compound according to claim 1 that is ethyl 1-hydroxy-3-((E)-3-tert-butyldimethylsilyloxy-7-phenylhept-1-en-1-yl) cyclohexane-1-acetate, and its enantiomers and diastereoisomers.

**6.** Compound according to claim 1 that is ethyl (1S*,3S*)-1-hydroxy-3-((E)-3-tert-butyldimethylsilyloxy-7-phenyl-hept-1-en-1-yl)cyclohexane-1-acetate, and its enantiomers and diastereoisomers.

**7.** Compound according to claim 1 that is ethyl (1S*,3S*)-1-hydroxy-3-((3R*S*,E)-3-hydroxy-7-phenyl-hept-1-en-1-yl) cyclohexane-1-acetate, and its enantiomers and diastereoisomers.

**8.** Process for the preparation of compounds of formula (I), characterised in that:
a compound of formula (II):

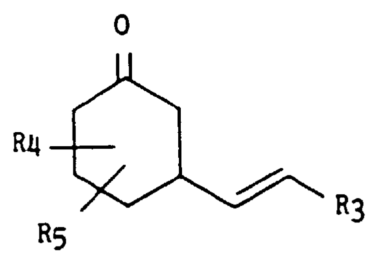

(II),

wherein $R_3$, $R_4$ and $R_5$ are as defined for formula (I), is reacted with a product of formula (III):

$$^{\ominus}B-R_2 \qquad (III),$$

wherein $R_2$ and B are as defined for formula (I), to obtain the corresponding compound of formula ($I_a$):

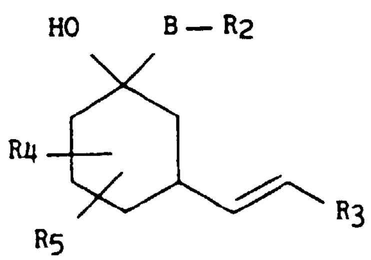

($I_a$)

wherein $R_2$, $R_3$, $R_4$, $R_5$ and B are as defined hereinbefore, which, if desired, may be:

- either subjected to the action of a product of formula (IV):

$$R'^{\oplus} \qquad (IV),$$

wherein R' represents an alkyl radical, to obtain the compounds of formula ($I_b$):

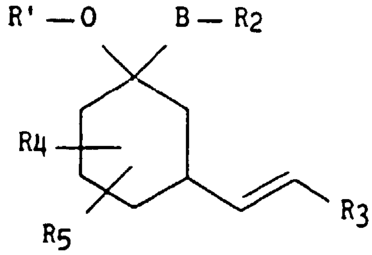

($I_b$)

wherein $R_2$, $R_3$, $R_4$, $R_5$, R' and B are as defined hereinbefore,
- or subjected to tosyl chloride, then to reduction with lithium aluminium hydride, to yield the compound of formula ($I_c$):

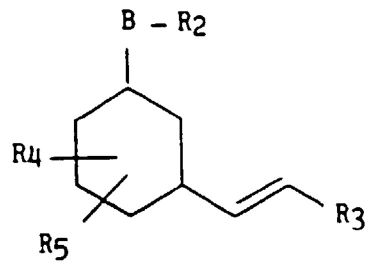

($I_c$)

wherein $R_2$, $R_3$, $R_4$, $R_5$ and B are as defined hereinbefore,
it being possible for the compounds of formulae (I/a), (I/b) and (I/c) in which $R_9$ represents a hydrogen atom and $R_7$ represents a hydroxyl radical to be oxidised to obtain the compounds of formula (I/d):

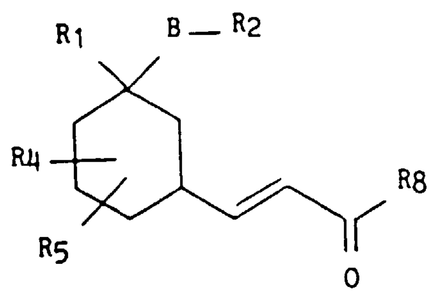

(I/d)

wherein $R_1$, $R_2$, $R_4$, $R_5$, $R_8$ and B are as defined hereinbefore,
the compounds of formulae (I/a), (I/b), (I/c) and (I/d) representing the totality of the compounds of formula (I), which compounds of formula (I) are, if desired, separated into their different enantiomers or diastereoisomers and, where appropriate, converted into their salts with a pharmaceutically acceptable acid or base.

**9.** Process according to claim 8, characterised in that the separation of the diastereoisomers is effected by means of column chromatography.

**10.** Process according to claim 8, characterised in that the separation of the diastereoisomers is effected by means of column chromatography and in that the group $R_7$ in the compounds of formula (I) according to claim 1 represents a tert-butyldimethylsilyloxy group.

**11.** Pharmaceutical composition comprising as active ingredient at least one compound according to claim 1 in combination with one or more pharmaceutically acceptable excipients or carriers.

**12.** Pharmaceutical composition according to claim 11 for use in the treatment and prevention of inflammatory disorders and pathological inflammatory conditions.

**13.** Pharmaceutical composition according to claim 11 for use in the treatment and prevention of chronic or acute, articular, pulmonary, cutaneous or renal inflammation, and especially in the prevention and treatment of arthritis, rheumatoid polyarthritis, osteoarthrosis, psoriasis, allergic disorders, asthma, inflammatory disorders of the intestine, gastro-intestinal ulcers, ischaemia, atherosclerosis, respiratory distress and septic shock.

**Patentansprüche**

**1.** Verbindungen der Formel (I):

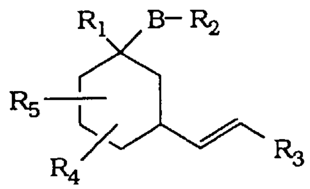

(I)

in der:

- B eine Alkylenkette mit 1 bis 8 Kohlenstoffatomen, die nichtsubstituiert oder durch 1 oder 2 Alkylgruppen substituiert ist,
- $R_1$ ein Wasserstoffatom oder eine aus Hydroxy und Alkoxy ausgewählte Gruppe.
- $R_2$ eine Gruppe ausgewählt aus:
  - $CH_2$-OH und

  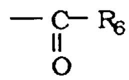

  in der $R_6$ eine Gruppe ausgewählt aus Hydroxy, Alkoxy und

  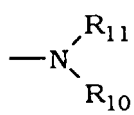

  darstellt, worin $R_{10}$ und $R_{11}$ jeweils unabhängig voneinander ein Wasserstoffatom, eine Alkylgruppe oder eine Aryl- oder Arylalkylgruppe darstellen, worin die Aryl- oder Arylalkylgruppe nichtsubstituiert oder am Arylkern durch eine oder mehrere Gruppen ausgewählt aus Halogen, Alkyl, Alkoxy und Trifluormethyl substituiert ist, oder $R_{10}$ und $R_{11}$ gemeinsam mit dem sie tragenden Stickstoffatom einen Heterocyclus ausgewählt aus Pyrrolidin, Piperidin, Piperazin, Morpholin und Thiomorpholin bilden;
- $R_3$ eine Gruppe der Formel:

  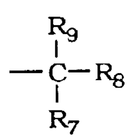

  in der:
- $R_7$ eine Gruppe ausgewählt aus Hydroxy, Alkoxy und -O-$R_A$ darstellt, worin $R_A$ eine Acylgruppe oder eine Schutzgruppe für die Hydroxylgruppe bedeutet,
- $R_9$ ein Wasserstoffatom oder eine Gruppe ausgewählt aus Alkyl, Aryl und Arylalkyl darstellt, wobei die Aryl- oder Arylalkylgruppe nichtsubstituiert oder am Arylkern durch eine oder mehrere Gruppen ausgewählt aus Halogen, Alkyl, Alkoxy und Trifluormethyl substituiert ist,
- oder $R_7$ und $R_9$ eine Oxogruppe bilden,
- und $R_8$ eine geradkettige oder verzweigte Alkylgruppe mit 1 bis 12 Kohlenstoffatomen, die nichtsubstituiert oder durch eine oder mehrere Gruppen ausgewählt aus:

  . Hydroxy,
  . Halogen,
  . nichtsubsituiertes Aryl,
  . Aryl substituiert durch eine oder mehrere Gruppen ausgewählt aus Halogen, Hydroxy, Alkyl, Alkoxy und

. Trifluormethyl,
. nichtsubstituiertes Cycloalkyl,
. Cycloalkyl substituiert durch eine oder mehrere Gruppen ausgewählt aus Halogen, Alkyl, Oxo und Alkoxy

darstellt,

- wobei $R_8$ gegebenenfalls in seinem Kohlenstoffgerüst:

  - eines oder zwei Heteroatome ausgewählt aus Stickstoff, Schwefel und Sauerstoff,
  - eine oder mehrere ungesättigte Bindungen aufweisen kann,

- $R_4$ und $R_5$ jeweils unabhängig voneinander eine aus Wasserstoff und Alkyl ausgewählte Gruppe oder $R_4$ und $R_5$ gemeinsam mit dem sie tragenden Cyclohexan eine 1,2,3,4-Tetrahydronaphthalingruppe oder eine Perhydronaphthalingruppe bedeuten, wobei bezüglich der Beschreibung der Formel (I), wenn nichts genaueres anderes angegeben ist:
- die Begriffe "Alkyl", "Alkoxy" und "Acyl" geradkettige oder verzweigte Gruppen mit 1 bis 6 Kohlenstoffatomen bedeuten,
- der Begriff "Aryl" für eine Phenyl- oder Naphthylgruppe steht,
- der Begriff "ungesättigte Bindung" für eine Doppelbindung oder eine Dreifachbindung steht und
- der Begriff "Cycloalkyl" für eine Gruppe mit 3 bis 7 Kohlenstoffatomen steht, deren Enantiomere und Diastereoisomere,

deren Z- und E-Isomere und
deren Additionssalze mit einer pharmazeutisch annehmbaren Säure oder Base.

**2.** Verbindungen nach Anspruch 1, worin $R_8$ eine nichtsubstituierte geradkettige oder verzweigte Alkylgruppe mit 1 bis 12 Kohlenstoffatomen bedeutet, deren Enantiomere und Diastereoisomere, deren Z- und E-Isomere und deren Additionssalze mit einer pharmazeutisch annehmbaren Säure oder Base.

**3.** Verbindungen nach Anspruch 1, worin $R_8$ eine geradkettige oder verzweigte Alkylgruppe mit 1 bis 12 Kohlenstoffatomen darstellt, die durch eine nichtsubstituierte oder eine substituierte Phenylgruppe substituiert ist, deren Enantiomere und Diastereoisomere, deren Z- und E-Isomere und deren Additionssalze mit einer pharmazeutisch annehmbaren Säure oder Base.

**4.** Verbindungen nach Anspruch 1, worin $R_7$ eine Hydroxylgruppe darstellt, deren Enantiomere und Diastereoisomere, deren Z- und E-Isomere und deren Additionssalze mit einer pharmazeutisch annehmbaren Säure oder Base.

**5.** Verbindung nach Anspruch 1, nämlich 1-Hydroxy-3-((E)-3-tert.-butyldimethylsilyloxy-7-phenyl-hept-1-en-1-yl)-cyclohexan-1-essigsäureethylester und dessen Enantiomere und Diastereoisomere.

**6.** Verbindung nach Anspruch 1, nämlich (1S*,3S*)-1-Hydroxy-3-((E)-3-tert.-butyldimethylsilyloxy-7-phenyl-hept-1-en-1-yl)-cyclohexan-1-essigsäureethylester und dessen Enantiomere und Diastereoisomere.

**7.** Verbindung nach Anspruch 1, nämlich (1S*,3S*)-1-Hydroxy-3-((3R*S*,E)-3-hydroxy-7-phenyl-hept-1-en-1-yl)-cyclohexan-1-essigsäureethylester und dessen Enantiomere und Diastereoisomere.

**8.** Verfahren zur Herstellung der Verbindungen der Formel (I), **dadurch gekennzeichnet,** daß man: eine Verbindung der Formel (II):

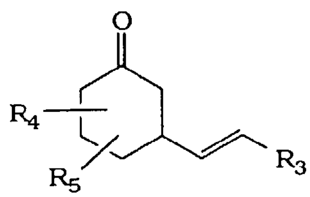

(II)

in der $R_3$, $R_4$ und $R_5$ die bezüglich der Formel (I) angegebenen Bedeutungen besitzen, mit einer Verbindung der Formel (III) umsetzt:

$^{\ominus}$ B-$R_2$ (III)

in der $R_2$ und B die bezüglich der Formel (I) angegebenen Bedeutungen besitzen, so daß man die entsprechende Verbindung der Formel (I/a) erhält:

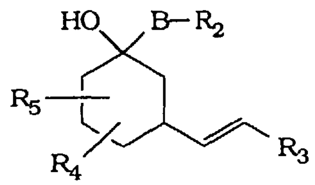

(I/a)

in der $R_2$, $R_3$, $R_4$, $R_5$ und B die oben angegebenen Bedeutungen besitzen, welche Verbindung man gewünschtenfalls,

- entweder der Einwirkung einer Verbindung der Formel (IV) unterwirft:

$R'^{\oplus}$ (IV)

in der R' eine Alkylgruppe darstellt, zur Bildung der Verbindungen der Formel (I/b):

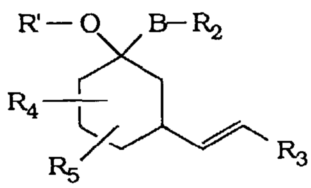

(I/b)

in der $R_2$, $R_3$, $R_4$, $R_5$, R' und B die oben angegebenen Bedeutungen besitzen,

- oder der Einwirkung von Tosylchlorid und dann einer Reduktion mit Lithiumaluminiumhydrid unterwirft zur Bildung der Verbindung der Formel (I/c):

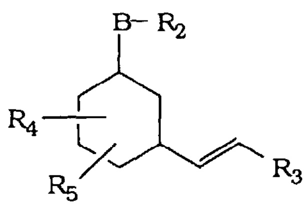

(I/c)

in der $R_2$, $R_3$, $R_4$, $R_5$ und B die oben angegebenen Bedeutungen besitzen,

welche Verbindungen der Formeln (I/a) (I/b) und (I/c), worin $R_9$ ein Wasserstoffatom und $R_7$ eine Hydroxylgruppe bedeuten, oxidiert werden können zur Bildung der Verbindungen der Formel (I/d):

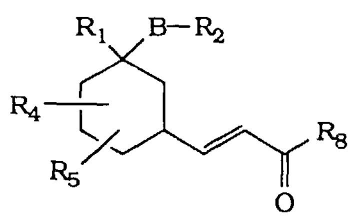

(I/d)

in der $R_1$, $R_2$, $R_4$, $R_5$, $R_8$ und B die oben angegebenen Bedeutungen besitzen, wobei die Verbindungen der Formeln (I/a), (I/b), (I/c) und (I/d) gemeinsam die Verbindungen der Formel (I) repräsentieren,
welche Verbindungen der Formel (I) gewünschtenfalls in ihre verschiedenen Enantiomeren oder Diastereoisomeren getrennt und gegebenenfalls mit einer pharmazeutisch annehmbaren Säure oder Base in die Salze überführt werden können,

**9.** Verfahren nach Anspruch 8, **dadurch gekennzeichnet,** daß die Trennung der Diastereoisomeren durch Säulenchromatographie erfolgt.

**10.** Verfahren nach Anspruch 8, **dadurch gekennzeichnet,** daß die Trennung der Diastereoisomeren durch Säulenchromatographie erfolgt und die Gruppe $R_7$ der Verbindungen der Formel (I) nach Anspruch 1 eine tert.-Butyldimethylsilyloxygruppe darstellt.

**11.** Pharmazeutische Zubereitung enthaltend als Wirkstoff mindestens eine Verbindung nach Anspruch 1 in Kombination mit einem oder mehreren pharmazeutisch annehmbaren Trägermaterialien oder Bindemitteln.

**12.** Pharmazeutische Zubereitung nach Anspruch 11 für die Behandlung und die Vorbeugung von Entzündungserkrankungen und pathologischen Entzündungszuständen.

**13.** Pharmazeutische Zubereitung nach Anspruch 11 zur Behandlung und zur Vorbeugung von chronischer oder akuter Gelenkentzündung, Lungenentzündung, Hautentzündung oder Nierenentzündung und insbesondere zur Vorbeugung und Behandlung der Arthritis, der rheumatoiden Polyarthritis, der Osteoarthrose, der Psoriasis, von allergischen Erkrankungen, des Asthmas, von Darmentzündungserkrankungen, von Magen-Darm-Geschwüren, der Ischämie, der Atherosklerose, der Atemnot und von septischem Schock.